(19) Europäisches Patentamt
European Patent Office
Office européen des brevets

(11) EP 1 444 517 B1

(12) # EUROPÄISCHE PATENTSCHRIFT

(45) Veröffentlichungstag und Bekanntmachung des Hinweises auf die Patenterteilung:
**27.06.2007 Patentblatt 2007/26**

(21) Anmeldenummer: **02787546.7**

(22) Anmeldetag: **04.11.2002**

(51) Int Cl.:
***G01N 33/542*** (2006.01) ***G01N 33/58*** (2006.01)
***C12Q 1/68*** (2006.01) ***C12Q 1/34*** (2006.01)
***C12Q 1/37*** (2006.01) ***C12Q 1/42*** (2006.01)
***C12Q 1/48*** (2006.01)

(86) Internationale Anmeldenummer:
**PCT/EP2002/012256**

(87) Internationale Veröffentlichungsnummer:
**WO 2003/040024 (15.05.2003 Gazette 2003/20)**

(54) **ASSAY BASIEREND AUF DOTIERTEN NANOTEILCHEN**
ASSAY BASED ON DOPED NANOPARTICLES
ANALYSE REPOSANT SUR DES NANOPARTICULES DOPEES

(84) Benannte Vertragsstaaten:
**AT BE BG CH CY CZ DE DK EE ES FI FR GB GR IE IT LI LU MC NL PT SE SK TR**

(30) Priorität: **05.11.2001 DE 10153829**

(43) Veröffentlichungstag der Anmeldung:
**11.08.2004 Patentblatt 2004/33**

(73) Patentinhaber: **Bayer Technology Services GmbH
51368 Leverkusen (DE)**

(72) Erfinder:
- **BOHMANN, Kerstin
50670 Köln (DE)**
- **HOHEISEL, Werner
51061 Köln (DE)**
- **KÖHLER, Burkhard
51373 Leverkusen (DE)**
- **DORN, Ingmar
50733 Köln (DE)**

(56) Entgegenhaltungen:
**WO-A-02/44725**
**WO-A-98/43072**
**US-A- 5 893 999**
**US-A- 6 159 686**
**US-B1- 6 207 392**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 444 517 B1

## Beschreibung

Die vorliegende Erfindung betrifft einen biotechnologischen Assay auf der Basis eines Resonanz Energie Transfers (RET), mit dem biologische Moleküle wie Enzyme, Antikörper, Nukleinsäure bindende Moleküle, Nukleinsäuren, Polynukleotide oder Morpholino nachgewiesen werden können.

Immunoassays oder Nukleinsäure-Nachweise sind Grundlage für viele Anwendungen in der medizinischen Diagnostik, bei der Produktionskontrolle von biotechnologisch hergestellten Produkten ebenso wie in der Forschung. Eine Methode, die hier verwendet wird, ist der Resonanz Energie Transfer (RET) zwischen Farbstoffen.

Das Prinzip des Resonanz Energie Transfers (RET) beruht auf einer strahlungslosen Übertragung von Energie von einem fluoreszenzfähigen Donor auf einen in räumlicher Nähe befindlichen Akzeptor. Mit dieser Technik können Entfernungen auf molekularer Ebene im Bereich zwischen ca. 1 und 8 nm bestimmt werden. Die auf den Akzeptor übertragene Energie kann einerseits durch interne Konversion strahlungslos relaxieren (RET) und führt dann lediglich zur Löschung (Quenchung) der Donor-Fluoreszenz. Zum anderen kann die übertragene Energie aber auch durch Fluoreszenz des Akzeptors abgegeben werden. In diesem Fall spricht man von Fluoreszenz Resonanz Energie Transfer (FRET). Diese Phänomene sind seit langer Zeit gut verstanden und im Falle einer Dipol-Dipol-Wechselwirkung zwischen Donor und Akzeptor durch die Theorie von Förster erklärt (siehe z. B. J. R. Lakowicz, "Principles of Fluorescence Spectroscopy", Kluwer Academic Press, New York, 1999, Seiten 368-445). Durch den Energietransfer wird die Intensität der Donor-Fluoreszenz sowie deren Zerfallszeit reduziert und die Fluoreszenz des Akzeptors entsprechend erhöht oder überhaupt erst angeregt bzw. sensibilisiert. Die Effizienz E des Energietransfers ist sehr empfindlich gegenüber dem Abstand R zwischen Donor und Akzeptor und fällt proportional zu $R_0^6/(R_0^6+R^6)$ ab. Die mittlere Reichweite des Energieübertrags, bei dem die Effizienz 50 % beträgt, wird durch eine materialspezifische Konstante, dem Försterradius $R_0$, bestimmt und liegt im Bereich weniger Nanometer (kleiner als 10 nm). Für den Fall, dass der angeregte Zustand des Akzeptors strahlungslos relaxiert, kommt es ausschließlich zu einer Reduzierung bis hin zur vollständigen Löschung der Donor-Fluoreszenz. Im folgenden wird im Fall, dass die Begriffe RET oder FRET alternativ verwendet werden können, der Begriff (F)RET benutzt. (F)RET-fähige Donor-Akzeptor-Paare werden als (F)RET-Paare bezeichnet. Im folgenden die Begriffe Fluoreszenz und Lumineszenz synonym verwendet.

In biologischen Systemen wird (F)RET dazu verwendet, die räumliche Nähe entsprechend markierter Biomoleküle bzw. Molekülgruppen zueinander nachzuweisen. Diese Methode kann als Nachweis von Protein-Protein-Wechselwirkungen z.B. als Nachweis der Antigen-Antikörper-Reaktion bei Immunreaktionen, einer Rezeptor-Liganden-Wechselwirkung, dem Nachweis einer Hybridisierung von Nukleinsäure oder der Bindung von Proteinen an Nukleinsäuren dienen. Der Nachweis selbst erfolgt über eine Messung der Intensitätsänderung bzw. der spektralen Änderung der Donor- oder Akzeptor-Fluoreszenz oder über die Messung einer Änderung der Zerfallszeit der Donorfluoreszenz. Zahlreiche Anwendungen hierzu sind in der Literatur beschrieben, wie der Nachweis von spezifischen Antigenen in Immunofluoreszenz-Assays (US 3 996 345; US 4 160 016; US 4 174 384; US 4 199 559), die Bestimmung des elektrostatischen Potentials in spezifischen, lokalisierten Bereichen auf der Oberfläche von Proteinen (Yamamoto et al.; J. Mol. Biol. 241, 1994, Seiten 714-731) oder das Verfahren des High-Throughput Screening (Boisclair et al.; J. of Biomolecular Screening 5, 2000, Seiten 319-328).

Es werden mit (F)RET Systemen auch absolute Distanzen zwischen zwei Molekülen oder innerhalb eines Biomoleküls bestimmt. Hierzu werden zwei Markierungen eingeführt werden, die in Abhängigkeit von ihrer Distanz zueinander, messbar miteinander wechselwirken. Bekannte Anwendungen dieser Methode sind die Protein- oder DNA-Strukturanalyse (Heyduk et al.; SPIE Vol. 3256, 1998, Seiten 218-222), die Bestimmung von Entfernungen innerhalb von Polypeptiden, (Lakowicz et al.; Biophys. Chem. 36, 1990, Seiten 99-115), Proteinen (K. Cai et al.; J. Biol. Chem. 271 1996, Seiten 27311-27320), Polynukleotiden (Hochstrasser et al.; Biophys. Chem. 45, 1992, Seiten 133-141 und Ozaki et al.; Nucl. Acids Res. 20, 1992, Seiten 5205-5214) oder anderer Makromoleküle, die Untersuchung von Membranen und Membranproteinen und deren Aufbau (S. Wang et al.; Biochemistry 27, 1988, Seiten 2033-2039), die Detektion (US 4 996 143; US 5 532 129; US 5 565 332) und Quantifizierung von amplifizierten Nukleinsäuren durch PCR (Polymerase Kettenreaktion) (US 5 538 848; US 5 723 591) z.B. für *in vitro* Diagnostik, genetische Analyse, Forensik, Lebens- und Agrarmitteltests oder Elternschaftstests. Die DNA oder RNA wird direkt, d.h. ohne zusätzliche Separierungsschritte, detektiert bzw. quantifiziert.

Eine quantitative Nukleinsäure-Bestimmung durch Echtzeit-PCR mit (F)RET Systemen ist der als TaqMan® Assay (Applied Biosystems Division der Perkin-Elmer Corp., Foster City, USA) bezeichnete 5'-Nuklease Assay (US 5 538 848; US 5 210 015; Holland et al.; Proc. Natl. Acad. Sci USA 88, 1991, Seiten 7276-7280; Lee et al.; Nuleic Acids Res. 21, 1993, Seiten 3761-3766). Auf einem ähnlichen Mechanismus beruht die Methode der Molecular Beacons (Tyagi und Kramer, Nature Biotechnology 14, 1996, Seiten 303-306; US 5 312 728).

Klassische, kommerziell erhältliche Materialien für effiziente (F)RET-Paare sind organische Farbstoff-Moleküle wie z.B. Fluorescein, Cyanin oder Rhodamin. Ein genereller Nachteil dieser organischen Fluoreszenzfarbstoffe ist, dass sie häufig eine für viele Anwendungen ungenügende Stabilität gegenüber einfallendem Licht aufweisen. Insbesondere in Anwesenheit von Sauerstoff oder Radikalen können sie zum Teil bereits nach einigen Millionen Lichtabsorptions-/

Lichtemissionszyklen irreversibel geschädigt oder zerstört werden. Weiterhin können die fluoreszierenden organischen Farbstoffmoleküle auch phototoxisch auf biologisches Material in der Umgebung wirken. Ungünstig für die gleichzeitige Auslesung mehrerer Farbstoffe, dem sogenannten "Multiplexing", wirken sich zum einen die breiten Emissionsbanden der organischen Fluoreszenzfarbstoffe mit ihren zusätzlichen Ausläufern in den langwelligen Bereich des Spektrums aus. Zum anderen ist deren üblicherweise geringe Stokes-Shift nachteilig, d.h. der Unterschied zwischen dem Anregungs- und Emissionsmaximum eines Farbstoffes sowie die relativ schmalen spektralen Anregungsbanden, innerhalb denen eine Anregung möglich ist. Dadurch müssen häufig mehrere Lichtquellen und/oder aufwendige Filtersysteme verwendet werden, was die gleichzeitige Auslesung mehrerer Farbstoffe zusätzlich einschränkt.

Als FRET-Paar können ebenfalls fluoreszierende Proteine eingesetzt werden. Der involvierte FRET-Prozess wird hier auch als Biolumineszenz-Resonanz-Energie-Transfer (BRET) bezeichnet. Dazu gehören das Grün Fluoreszierende Protein GFP (US 5 491 084) und dessen Variationen, die andere Absorptions- bzw. Emissionsmaxima besitzen, wie z.B. Gelb (YFP) oder Cyan (CFP) Fluoreszierende Proteine (US 5 625 048). GFPs können dabei entweder als Donor und Akzeptor oder in Kombination mit einem anderen Fluorophore wie z.B. Fluorescein oder Luciferase eingesetzt werden (Übersichtsartikel: Pollok and Heim; Trends Cell Biol. 9, 1999, Seiten 57-60). Problematisch ist die geringe Auswahl an verschiedenen GFP Proteinen, die den Ansprüchen für ein geeignetes FRET-Paar genügen (ausreichend großer Unterschied der Anregungswellenlängen, ausreichende Überlappung der Emmissions- und Anregungswellenlängen von Donor und Akzeptor). So konnten bisher lediglich zwei Kombinationen von GFPs als FRET-Paar erfolgreich angewendet werden (Übersichtsartikel: Pollok and Heim; Trends Cell Biol. 9, 1999, Seiten 57-60). Auch in Kombination mit anderen Farbstoffen oder bioluminiszenten Proteinen ist die geringe Anzahl und stark unterschiedliche Intensität der GFPs ein limitierender Faktor

Als eine Alternative zu organischen Farbstoffen werden auch Metallchelate bzw. Metallkomplexe für FRET benutzt (siehe z. B. Selvin; IEEE J. of Selected Topics in Quantum Electronics 2, 1996, Seiten 1077-1087).

Lanthanid-Chelate können sowohl als (F)RET-Paare eingesetzt werden (Clark et al., Anal. Biochem. 210, 1993, Seiten 1 ff.), als auch nur als Donor, der die Energie auf organische Fluoreszenzfarbstoffe (Thomas et al.; Proc. Natl. Acad. Sci. 75, 1978, Seiten 5746 ff; S. G. Jones et al.; Journal of Fluorescence 11, 2001, Seiten 13-21) oder auf Quencher (Tanaka et al.; J. Photochem. Photobiol. A 74, 1993, Seiten 15 ff; Matko et al.; Biochemistry 31, 1992, Seiten 703 ff.), überträgt.

Systeme bzw. Assays, die auf Energietransfer und auf Fluorochrome und Chelate mit langer Lebensdauer basieren, sind in einer Reihe von Patenten offenbart worden (WO 8 707 955, EP 242 527, EP 439 036, WO 9201225, US 4 822 733, US 5 279 943, US 5 622 821, US 5 656 433, US 5 998 146, US 6 239 271). Sie benutzen Time Gated Fluoremetry (TGF) und/oder Time Resolved Fluoremetry (TRF) zum Nachweis eines Analyten.

Unter TGF versteht man dabei einen Messmodus, bei dem die Anregung mit einer gepulsten Lichtquelle (Laser, Blitzlampe) erfolgt und nach einer dann folgenden definierten Verzögerungszeit die Lichtemission innerhalb eines bestimmten Zeitfensters gemessen wird. Die Verzögerungszeit ist lang genug, um mit genügend hoher Intensität die langlebige Fluoreszenz der Lanthanid-Chelate nachzuweisen. Die kurzlebige Hintergrundfluoreszenz (meist < 1 $\mu$s), hervorgerufen durch intrinsische Autofluoreszenz des biologischen Materials, Verunreinigungen im Lösemittel oder umschließender Gefäßmaterialien, wird durch die Verzögerungszeit allerdings nahezu vollständig diskriminiert. Im Unterschied zum TGF-Modus wird bei Messungen im TRF-Modus die Fluoreszenz als Funktion der Zeit bei fester Wellenlänge gemessen. Dabei wird der Donor ebenfalls durch eine gepulste oder aber auch durch eine andersartig modulierte Lichtquelle angeregt.

Nachteilig an den Lanthanid-Chelaten oder Metallkomplexe ist jedoch ihre für manche Anwendungen geringe chemische Stabilität oder die Abhängigkeit der Fluoreszenzeigenschaften von der chemischen Umgebung der Teilchen. Oft sind auch zusätzliche Separationsschritte oder eine zusätzliche Komplexierung notwendig, um eine Fluoreszenz messen zu können.

FRET-Effekte bei partikulären Label-Systemen die auf Halbleiter-Nanokristallen, den sogenannten Quantum Dots, beruhen, wurden ebenfalls beobachtet: (Bawendi et al. und C. Kagan et al.; Phys. Rev. Lett. 76, 1996, Seiten 1517-1520). Quantum Dots können auch mit organischen Fluorophoren in Wechselwirkung treten (O. Schmelz et al.; Langmuir 17, 2001, Seiten 2861-2865).

Es können FRET-Effekte zwischen Quantum Dots untereinander oder auch zwischen Quantum Dots und anderen Substanzen (z.B. Farbstoffe) ausgenutzt werden. In WO 00/29617 wird offenbart, dass Proteine und Nukleinsäuren mit Hilfe von Quantum Dots als Marker nachgewiesen werden können. Insbesondere wird auch ihr Einsatz als Fluorophor bei den als "Molecular Beacon" bekannten haarnadelartige DNA-Strukturen offenbart.

US B1 6207392 offenbart die Verwendung von Quantum Dots, d.h Halbleiter-Nanoteilchen, ohne Dotierung in FRET-Assays. In der vorliegenden Erfindung werden lumineszierende anorganische dotierte Nanoteilchen (lad-Nanoteilchen) beansprucht.

Nachteilig an den Quantum Dots ist jedoch, dass sie mit höchster Präzision hergestellt werden müssen. Da die Emissionswellenlänge des Fluoreszenzlichts von der Größe der Quantum Dots abhängt, muss in einer Probe eine sehr enge Größenverteilung der Teilchen erreicht werden. Um die für das Multiplexing erforderliche schmale Bandbreite

des Fluoreszenzlichts zu gewährleisten, dürfen die Größenunterschiede zwischen Quantum Dots einer Sorte nur wenige Angström, das heißt nur wenige Monolagen betragen. Dies stellt hohe Anforderungen an die Synthese. Außerdem weisen Quantum Dots im Normalfall bedingt durch strahlungslose Elektron-Loch-Paar Rekombinationen auf deren Oberfläche relativ schwache Quanteneffizienzen auf. Aus diesem Grund müssen zur Erhöhung der Quanteneffizienzen Kern-Hüllen-Strukturen erzeugt werden (Xiaogang Peng et al.; J. Am. Chem. Soc. 119, 1997, Seiten 7019-7029), für die eine aufwändigere Synthese notwendig ist.

Die Zerfallszeit der Fluoreszenz ist bei Quantum Dots zudem sehr kurzlebig und liegt im unteren Nanosekundenbereich. Deshalb sind keine Messungen im TGF-Modus und nur unter größerem technisch-apparativem Aufwand Messungen im TRF-Modus möglich. Ein weiterer Nachteil der Quantum Dot Systeme ist deren Zusammensetzung, die vielfach toxische Elemente wie z. B. Cadmium, Selen oder Arsen enthalten.

Nanoskalige Phosphore mit einer Größe von unter 50 nm, die im folgenden als lumineszierende anorganische dotierte Nanoteilchen (lad-Nanoteilchen) bezeichnet werden, sind in wissenschaftlichen Publikationen vielfach beschrieben worden.

Die publizierten lad-Nanoteilchen bestehen aus Oxiden, Sulfiden, Phosphaten oder Vanadaten, die mit Lanthaniden oder aber mit Mn, Al, Ag oder Cu dotiert sind. Diese lad-Nanoteilchen fluoreszieren auf Grund ihrer Dotierung in einem engen Spektralbereich. Unter anderem ist die Herstellung der folgenden lad-Nanoteilchen publiziert worden: $LaPO_4$:Ce,Tb; (K. Riwotzki et al.; Angewandte Chemie, Int. Ed. 40, 2001, Seiten 573-576); $YVO_4$:Eu, $YVO_4$:Sm, $YVO_4$:Dy (K. Riwotzki, M. Haase; Journal of Physical Chemistry B; Vol. 102, 1998, Seiten 10129-10135); $LaPO_4$:Eu, $LaPO_4$:Ce, $LaPO_4$:Ce,Tb; (H. Meyssamy, K. Riwotzki, A. Kornowski, S. Naused, M. Haase; Advanced Materials, Vol. 11, Issue 10, 1999, Seiten 840-844); (K. Riwotzki, H. Meyssamy, A. Kornowski, M. Haase; Journal of Physical Chemistry B Vol. 104, 2000, Seiten 2824-2828); ZnS:Tb, ZnS:$TbF_3$, ZnS:Eu, ZnS:$EuF_3$, (M. Ihara, T. Igarashi, T. Kusunoki, K. Ohno; Society for Information Display, Proceedings 1999, Session 49.3); $Y_2O_3$:Eu (Q. Li, L. Gao, D. S. Yan; Nanostructured Materials Vol. 8, 1999, Seiten 825 ff); $Y_2SiO_5$:Eu (M. Yin, W. Zhang, S. Xia, J. C. Krupa; Journal of Luminescence, Vol. 68, 1996, Seiten 335 ff.); $SiO_2$:Dy, $SiO_2$:Al, (Y. H. Li, C. M. Mo, L. D. Zhang, R. C. Liu, Y. S. Liu; Nanostructured Materials Vol. 11, Issue 3, 1999, Seiten 307-310); $Y_2O_3$:Tb (Y. L. Soo, S. W. Huang, Z. H. Ming, Y. H. Kao, G. C. Smith, E. Goldburt, R. Hodel, B. Kulkarni, J. V. D. Veliadis, R. N. Bhargava; Journal of Applied Physics Vol. 83, Issue 10, 1998, Seiten 5404-5409); CdS:Mn (R. N. Bhargava, D. Gallagher, X. Hong, A. Nurmikko; Physical Review Letters Vol. 72, 1994, Seiten 416-419); ZnS:Tb (R. N. Bhargava, D. Gallagher, T. Welker; Journal of Luminescence, Vol. 60, 1994, Seiten 275 ff.). Einen Überblick über die bekannten lumineszierenden anorganischen dotierten Materialien, die eine Größe von einigen Mikrometern haben und deren Verwendung als technische Phosphore, gibt Ullmann's Encyclopedia of Industrial Chemistry, WILEY-VCH, 6th edition, 2001 Electronic Release, Kapitel "Luminescent Materials: 1. Inorganic Phosphors".

Für die Lichtemission, die in Leuchtphosphoren, wie sie z.B. für Leuchtstofflampen verwendet werden, hervorgerufen wird, ist in vielen Fällen ebenfalls ein (F)RET zwischen einem Donor (Sensitizer) und einem Akzeptor (Emitter) verantwortlich (D. Dexter; J. Chem. Phys. 21, 1953, Seiten 836-850, T. Jüstel et al.; Angewandte Chemie, International Edition 37, 1998, Seiten: 3084-3103). Da jedoch bei einem Leuchtphosphor Donor und Akzeptor in einem gemeinsamen Kristallgitter vorliegen, kann das (F)RET-System der Leuchtphosphore nicht als Nachweis für eine Parameteränderung durch einen biochemischen Vorgangs dienen.

In US 5 043 265 wird offenbart, dass biologische Makromoleküle, die an nanoskalige Leuchtphosphorteilchen gekoppelt sind, durch Messung der Fluoreszenz nachgewiesen werden können. Es wird ausgeführt, dass die Fluoreszenz der Teilchen mit kleiner werdendem Durchmesser rasch an Intensität verliert und die Teilchen deshalb größer als 20 nm und bevorzugt sogar größer als 100 nm sein sollten.

Für den Einsatz als biologische Label werden spezielle Typen von Leuchtphosphoren in US 5,674,698 offenbart. Es handelt sich dabei um "Aufkonvertierende Phosphore" (upconverting phosphors), die die Eigenschaft haben, über einen Zwei-Photonen-Prozess Licht zu emittieren, das eine kleinere Wellenlänge besitzt als das absorbierte Licht. Durch Verwendung dieser Teilchen kann quasi hintergrundfrei gearbeitet werden, da solche Autofluoreszenz weitestgehend unterdrückt ist. Die Teilchen werden durch Mahlung und anschließender Temperung hergestellt. Die Teilchengröße liegt zwischen 10 nm und 3 μm, bevorzugt zwischen 300 nm und 1 μm. Verwendet werden diese Teilchen in erster Linie in Immunoassays (siehe z. B. Niedbala et al.; Analytical Biochemistry 293, 2001, Seiten 22-30). Nachteilig an diesen Teilchen ist einerseits die durch den Herstellungsprozess bedingte breite Größenverteilung. Zum anderen gibt es bei den kleineren Teilchen, bedingt durch die Herstellung, häufig qualitative Einschränkungen, die sich in der bevorzugten Teilchengröße von 300 nm - 1 μm widerspiegelt. Im allgemeinen ist für die Anregung eines Zwei-Photonen-Prozess bei vergleichbarer Emissionsintensität eine höhere Anregungsintensität notwendig als beim Ein-Photonen-Prozess.

WO A 98/43072 offenbart ein (F)RET Assay, wobei die Donoren dotierte Phosphore sind, die mit Anti-Stokes-Verschiebung (=up-converting) emittieren, und eine amorphe oder kristalline Struktur sowie eine Größe von 10 - 100 nm aufweisen. Beschrieben wird nur eine Mischung von Ytterbium und Erbium Ionen in einer amorphen Matrix. Überaschenderweise fluoreszieren kristalline Nanoteilchen deutlich besser als amorphe. Die vorliegende Erfindung betrifft daher kristalline Nanoteilchen.

US A 5 893 999 offenbart Teilchen mit Größen 1 nm - 1 µm, bevorzugt 1 nm - 100 nm zur Verwendung als Biolabel geht aber nicht auf (F)RET-Assays ein.

US A 6 159 686 offenbart Phosphore als Label in FRET-Assays, die kristallin sind und deren Größe bei < 2 µm, bevorzugt < 1µm, besonders bevorzugt 0,1 - 0,3 µm "oder kleiner liegt. Teilchen dieser Größe neigen zur hohen Sedimentation und zeigen eine niedrige Diffusionsgeschwindigkeit, was für Assays von erheblichem Nachteil ist.

Zur Durchführung von photophysikalischer Katalyse oder photodynamischer Therapie werden in US 6 159 686 spezielle Phosphor-Farbstoff-Komplexe offenbart. Aufkonvertierende Phosphore, werden zunächst mit unschädlichem infrarotem Licht angeregt. Energie im Bereich des sichtbaren Lichtes wird dann auf den Farbstoff übertragen, welcher wiederum als Katalysator wirkend seine Energie auf ein Zielmolekül überträgt. Mit solchen Paaren aus aufkonvertierenden Phosphoren und geeigneten Farbstoffen lassen sich auch Zielanalyten nachweisen. Dazu wird bei Anwesenheit des Zielanalyten ein Komplex aus Zielanalyt, Phosphor und Farbstoff gebildet, der dann durch die räumliche Nähe einen Energietransfer vom Phosphor auf den Farbstoff zulässt. Ebenso ist in diesem Patent die Verwendung dieser aufkonvertierenden Phosphore mit einem entsprechendem "matched label" in homogenen, heterogenen und kompetitiven Assays offenbart. Dabei können die Phosphore sowohl als Donor als auch als Akzeptor eingesetzt werden.

Die erfindungsgemäße Aufgabe besteht in der Bereitstellung eines Assays zum Nachweis eines biologischen Zielmoleküls, das die im Stand der Technik beschriebenen Nachteile nicht aufweist.

Die Aufgabe wird erfindungsgemäß durch einen auf Resonanz Energie Transfer (RET) oder auf Fluoreszenz Resonanz Energie Transfer (FRET) basierenden Assay gelöst, der eine erste Molekülgruppe A, die mit mindestens einem erfindungsgemäßem Energie-Donor markiert ist, und mindestens eine zweite Molekülgruppe B, die jeweils mit mindestens einem Energie-Akzeptor markiert ist, enthält.

Unter einem Donor im Sinne der Erfindung wird ein Molekül oder Teilchen verstanden, das durch eine äußere Strahlungsquelle (elektromagnetische Strahlung oder Teilchenstrahlung) kontinuierlich oder zeitlich moduliert energetisch angeregt wird und fluoreszenzfähig ist.

Unter einem Akzeptor im Sinne der Erfindung wird ein Molekül oder Teilchen verstanden, das durch Energietransfer über den Donor angeregt wird, die Donor-Fluoreszenz ganz oder teilweise löscht und selbst fluoreszenzfähig sein kann, aber nicht sein muss. Ein nicht fluoreszenzfähiger Donor relaxiert strahlungslos.

Donor und/oder Akzeptor umfassen erfindungsgemäß lad-Nanoteilchen mit einer Ausdehnung von ≤ 10 Nanometer, die nach einer energetischen Anregung elektromagnetische Strahlung mit einer Stokes- oder Antistokes-Verschiebung emittieren.

Der Vorteil von einem Assay basierend auf lad-Nanoteilchen mit einer Ausdehnung von 10 Nanometern oder kleiner ist, dass sie ein geringeres Potential für sterische Probleme oder unerwünschte Sedimentation in einem Assay aufweisen, als dies bei größeren Teilchen der Fall sein kann. Außerdem wird die Kinetik einer Bindungsreaktion (z. B. Immunreaktion oder DNA-Hybridisierung) oder eines zu untersuchenden biochemischen Vorgangs durch die Anwesenheit des lad-Nanoteilchens weniger beeinflußt.

Darüber hinaus weisen größere lad-Teilchen Nachteile bei Messungen im TRF-Modus auf. Bei einem (F)RET ist der Energieübertag von beispielsweise einem als Donor wirkenden lad-Teilchen auf ein in räumlicher Nähe befindliches und als Akzeptor wirkendes Molekül nur innerhalb eines wenige Nanometer reichenden Abstands möglich. Dies bedeutet, dass bei größeren Teilchen ein bedeutender Teil des Teilchenvolumens und somit der Dotierungsionen im Teilchen nicht in Reichweite zu dem vor der Teilchenoberfläche befindlichen Akzeptor sind und deshalb nicht am (F)RET beteiligt sind. Dadurch wird der Effekt der durch einen (F)RET verursachten Änderung der Zerfallszeit (TRF-Modus) weniger ausgeprägt oder unter Umständen nicht mehr meßbar. Aus den gleichen Gründen wäre auch eine vollständige oder zumindest bedeutende Löschung der Donorfluoreszenz durch den Akzeptor nicht mehr möglich.

Ein RET oder FRET kann durch eine Dipol-Dipol-Wechselwirkung (Förster-Transfer), durch Wechselwirkung unter Beteiligung höherer Multipole oder durch Migration von Ladungen oder Excitonen erfolgen. Bei einem Förster-Transfer muss die spektrale Überlappung zwischen Donoremission uns Akzeptorabsorption hinreichend groß sein. Da die Effizienz des Energietransfers abhängig von der Entfernung ist, kann somit der Abstand zwischen Donor und Akzeptor gemessen werden.

Bevorzugt ist mindestens einer der beiden Partner (Donor oder Akzeptor) ein lumineszierendes anorganisches dotiertes Nanoteilchen mit langer Zerfallszeit der Fluoreszenz (> 5 ns). Der entsprechend andere Partner enthält entweder einen molekularen, organischen Chromophor oder ein lumineszierendes anorganisches dotiertes Nanoteilchen, das vorzugsweise eine kürzere Zerfallszeit der Fluoreszenz aufweist. Das lad-Nanoteilchen mit langer Zerfallszeit der Fluoreszenz weist dabei eine Halbwertszeit von mehr als 5 ns, bevorzugt zwischen 1 µs und 50 ms und besonders bevorzugt zwischen 100 µs und 10 ms auf. In einem Assay dieser Ausführungsform kann der Donor mit einer gepulsten Lichtquelle geeigneter Wellenlänge angeregt werden. Bei entsprechender räumlicher Nähe des Donor-Akzeptor-Paares zueinander (wenige Nanometer) kann nun ein FRET stattfinden, d.h. der Akzeptor z.B. ein molekulares Chromophor wird sensibilisiert und kann seine Energie durch Lichtemission abgeben. Da die Zerfallszeit z.B. der Donorfluoreszenz sehr lang ist, ist wegen des FRET auch die Zerfallszeit der sensibilisierten Fluoreszenz des Akzeptors sehr lang und somit sehr viel länger als durch eine direkte Anregung des Akzeptors durch die gepulste Lichtquelle. Mit einer Messung der Fluoreszenz

im TGF-Modus kann deshalb durch Ausblenden der kurzlebigen Akzeptor-Fluoreszenz nahezu untergrundfrei und somit mit hoher Sensitivität die sensibilisierte Akzeptor-Fluoreszenz nachgewiesen werden. Bei Verwendung einer mit einer geeigneten Frequenz modulierten Lichtquelle können auch phasensensitive Messungen durchgeführt werden. Auch der Donor kann eine kurzlebige und der Akzeptor eine langlebige Fluoreszenz aufweisen, wie dies z.B an dem System von dotierten $LaPO_4$-Nanoteilchen beobachtet werden kann. Hier wirken solche mit Cer-Ionen dotierten Nanoteilchen als Donor und solche mit Terbium-Ionen dotierten als Akzeptor.

In einer weiteren Auführungsform besteht der Donor aus einem lad-Nanoteilchen und der Akzeptor aus einem leitfähigen Material. Solche Materialien können Metalle wie z.B. Gold (Au), Silber (Ag), Platin (Pt) oder leitfähige Oxide, wie z.B. Indium-Zinn-Oxid (ITO) oder leitfähige Polymere sein. Sie können dabei in partikulärer Form als Nano- oder Mikroteilchen vorliegen oder aus einer ebenen Oberfläche, die auch strukturiert sein kann, bestehen.

Die in dem erfindungsgemäßen Assay eingesetzten lad-Nanoteilchen sind mit Fremdionen derart dotiert, dass der durch schmal- oder breitbandige, gepulste, modulierte oder kontinuierliche elektromagnetische Strahlung mit Wellenlängen im Bereich des infraroten Lichts, des sichtbaren Lichts, des UV, des Röntgenlichts oder der $\gamma$-Strahlung oder Teilchenstrahlung wie Elektronenstrahlung oder durch einen Teilchenstrahl angeregt werden kann und der Akzeptor durch zeitaufgelöste oder kontinuierliche Messung von materialspezifischem Fluoreszenzlicht bzw. dessen Änderung qualitativ und/oder quantitativ nachgewiesen werden kann.

Der Nachweis der biochemischen Reaktion erfolgt durch Messung eines RET oder FRET, das heißt durch Messung der Änderung der Lumineszenzeigenschaften (Intensität, spektral oder durch Veränderung der Zerfallszeit) der lad-Nanoteilchen und/oder der anderen beteiligten Chromophore. Auf diese Weise lassen sich die Abstandsänderungen der beteiligten (F)RET Partner in einem Assay nachweisen.

Über die Veränderung der Zerfallszeit des Donors kann die räumliche Nähe eines Akzeptors in einem (F)RET System nachgewiesen werden. Aufgrund der Präsenz eines weiteren Zerfallskanals durch den Energietransfer zum Akzeptor wird die Zerfallszeit der Donor-Fluoreszenz signifikant verkürzt. Diese Änderung kann sowohl bei der Donor-Fluoreszenz als auch bei der sensibilisierten Akzeptor-Fluoreszenz gemessen werden (Messung im TRF-Modus). Die Emissionszerfallszeit als Beobachtungsgröße für FRET bietet eine Alternative zur Messung von Intensitäten. Sie macht eine Messung unabhängig von Konzentrationseffekten, Quanteneffizienz des Chromophors, unvollständigem Labeling und teilweiser oder vollständiger Löschung der Akzeptor-Fluoreszenz. Nahezu jedes nachgewiesene Photon ist ein Beitrag zum Nutzsignal. Aus der Verringerung der Zerfallszeit der Donor-Fluoreszenz kann bei einem Förster-Transfer unter Verwendung der hierzu bekannten mathematischen Beziehungen auch auf die räumliche Entfernung zwischen Donor und Akzeptor geschlossen werden. Ein wichtiger Vorteil bei der Verwendung von lad-Nanoteilchen ist deren intrinsisch lange Zerfallszeit, die oft bis in den Bereich von einigen Millisekunden reicht und deshalb mit einfachen experimentellen Mitteln bequem zu erfassen ist.

Die lad-Nanoteilchen haben eine nahezu sphärische Morphologie mit Ausdehnungen im Bereich von 1 nm bis 10 nm und bevorzugt im Bereich von 2 nm bis 10 nm. Unter Ausdehnungen wird der maximale Abstand von zwei auf der Oberfläche eines lad-Nanoteilchens liegenden Punkte verstanden. Die lad-Nanoteilchen können auch eine ellipsoidförmige Morphologie besitzen oder facetiert sein mit Ausdehnungen, die in den oben angegeben Grenzen liegen.

Die Teilchengröße kann mit der Methode der Ultrazentrifugation, der Gelpermeationschromatographie oder mittels Elektronenmikroskopie bestimmt werden.

Im Sinne der Erfindung geeignete Materialien für die lad-Nanoteilchen sind anorganische Nanokristalle, deren Kristallgitter (Wirtsmaterial) mit Fremdionen dotiert sind. Hierunter zählen insbesondere alle Materialien und Materialklassen, die als sogenannte Phosphore z. B. in Leuchtschirmen (z. B. für Elektronenstrahlröhren) oder als Beschichtungsmaterial in Fluoreszenzlampen (für Gasentladungslampen) Verwendung finden, wie sie zum Beispiel in Ullmann's Encyclopedia of Industrial Chemistry, WILEY-VCH, 6th edition, 2001 Electronic Release, Kapitel "Luminescent Materials: 1. Inorganic Phosphors" genannt sind, sowie die aus dem oben zitierten Stand der Technik bekannten lad-Nanoteilchen. In diesen Materialien dienen die Fremdionen als Aktivatoren für die Emission von Fluoreszenzlicht nach Anregung durch UV-, sichtbares oder IR-Licht, Röntgen- oder Gammastrahlen oder Elektronenstrahlen. Bei einigen Materialien werden auch mehrere Sorten von Fremdionen in das Wirtsgitter eingebaut, um einerseits Aktivatoren für die Emission zu erzeugen und um andererseits die Anregung des Teilchensystems effizienter zu gestalten oder um die Absorptionswellenlänge durch Verschiebung an die Wellenlänge einer gegebenen Anregungslichtquelle anzupassen (sogenannte Sensitizer). Der Einbau mehrerer Sorten von Fremdionen kann auch dazu dienen, gezielt eine bestimmte Kombination von Fluoresezenzbanden, die von einem Teilchen emittiert werden sollen, einzustellen.

Das Wirtsmaterial der lad-Nanoteilchen besteht vorzugsweise aus Verbindungen des Typs XY. Dabei ist X ein Kation aus Elementen der Hauptgruppen 1a, 2a, 3a, 4a der Nebengruppen 2b, 3b, 4b, 5b, 6b, 7b oder der Lanthaniden des Periodensystems. In einigen Fällen kann X auch eine Kombination bzw. Mischung aus den genannten Elementen sein. Y kann ein mehratomiges Anion, enthaltend ein oder mehrere Element(e) der Hauptgruppen 3a, 4a, 5a, der Nebengruppen 3b, 4b, 5b, 6b, 7b und/oder 8b sowie Elemente der Hauptgruppen 6a und/oder 7a, sein. Y kann aber auch ein einatomiges Anion aus der Hauptgruppe 5a, 6a oder 7a des Periodensystems sein. Das Wirtsmaterial der lad-Nanoteilchen kann auch aus einem Element der Hauptgruppe 4a des Periodensystems bestehen. Als Dotierung können

Elemente der Hauptgruppen 1 a, 2a oder aus der Gruppe enthaltend Al, Cr, Tl, Mn, Ag, Cu, As, Nb, Nd, Ni, Ti, In, Sb, Ga, Si, Pb, Bi, Zn, Co und/oder Elemente der Lanthaniden dienen. Auch Kombinationen von zwei oder mehreren dieser Elemente können in unterschiedlichen relativen Konzentrationen zueinander als Dotierungsmaterial dienen. Die Konzentration des Dotierungsmaterials im Wirtsgitter beträgt zwischen $10^{-5}$ mol-% und 50 mol-%, bevorzugt zwischen 0,01 mol-% und 30 mol-%, besonders bevorzugt zwischen 0,1 mol-% und 20 mol-%. Das Dotierungsmaterial wird so gewählt, dass die Zerfallszeit der durch es induzierten Fluoreszenz lang ist (> 100 ns).

Bevorzugt werden Sulfide, Selenide, Sulfoselenide, Oxysulfide, Borate, Aluminate, Gallate, Silikate, Germanate, Phosphate, Halophosphate, Oxide, Arsenate, Vanadate, Niobate, Tantalate, Sulfate, Wolframate, Molybdate, Alkalihalogenide sowie andere Halogenide oder Nitride als Wirtsmaterialien für die lad-Nanoteilchen verwendet. Beispiele für diese Materialklassen sind zusammen mit den entsprechenden Dotierungen in der folgenden Liste angegeben (Materialien des Typs B:A mit B = Wirtsmaterial und A = Dotierungsmaterial):

LiI:Eu; NaI:Tl; CsI:Tl; CsI:Na; LiF:Mg; LiF:Mg,Ti; LiF:Mg,Na; $KMgF_3$:Mn; $Al_2O_3$:Eu; BaFCl:Eu; BaFCl:Sm; BaFBr:Eu; $BaFCl_{0,5}Br_{0,5}$:Sm; $BaY_2F_8$:A (A = Pr, Tm, Er, Ce); $BaSi_2O_5$:Pb; $BaMg_2Al_{16}O_{27}$:Eu; $BaMgAl_{14}O_{23}$:Eu; $BaMgAl_{10}O_{17}$:Eu; $BaMgAl_2O_3$:Eu; $Ba_2P_2O_7$:Ti; $(Ba,Zn,Mg)_3Si_2O_7$:Pb; $Ce(Mg,Ba)Al_{11}O_{19;}$ $Ce_{0,65}Tb_{0,35}MgAl_{11}O_{19:Ce,Tb;}$ $MgAl_{11}O_{19}$:Ce,Tb; $MgF_2$:Mn; MgS:Eu; MgS:Ce; MgS:Sm; MgS:(Sm,Ce); (Mg,Ca)S:Eu; $MgSiO_3$:Mn; $3,5MgO{\cdot}0,5MgF_2{\cdot}GeO_2$:Mn; $MgWO_4$:Sm; $MgWO_4$:Pb; $6MgO{\cdot}As_2O_5$:Mn; $(Zn,Mg)F_2$:Mn; $(Zn_4Be)SO_4$:Mn; $Zn_2SiO_4$:Mn; $Zn_2SiO_4$:Mn,As; ZnO:Zn; ZnO:Zn,Si,Ga; $Zn_3(PO_4)_2$:Mn; ZnS:A (A = Ag, Al, Cu); (Zn,Cd)S:A (A = Cu, Al, Ag, Ni); $CdBO_4$:Mn; $CaF_2$:Mn; $CaF_2$:Dy; CaS:A (A = Lanthanide, Bi); (Ca,Sr)S:Bi; $CaWO_4$:Pb; $CaWO_4$:Sm; $CaSO_4$:A (A = Mn, Lanthanide); $3Ca_3(PO_4)_2{\cdot}Ca(F,Cl)_2$:Sb,$M_n$; $CaSiO_3$:Mn,Pb; $Ca_2Al_2Si_2O_7$:Ce; $(Ca,Mg)SiO_3$:Ce; $(Ca,Mg)SiO_3$:Ti; $2SrO{\cdot}6(B_2O_3){\cdot}SrF_2$:Eu; $3Sr_3(PO_4)_2{\cdot}CaCl_2$:Eu; $A_3(PO_4)_2{\cdot}ACl_2$:Eu (A = Sr, Ca, Ba); $(Sr,Mg)_2P_2O_7$:Eu; $(Sr,Mg)_3(PO_4)_2$:Sn; SrS:Ce; SrS:Sm,Ce; SrS:Sm; SrS:Eu; SrS:Eu,Sm; SrS:Cu,Ag; $Sr_2P_2O_7$:Sn; $Sr_2P_2O_7$:Eu; $Sr_4Al_{14}O_{25}$:Eu; $SrGa_2S_4$:A (A = Lanthanide, Pb); $SrGa_2S_4$:Pb; $Sr_3Gd_2Si_6O_{18}$:Pb,Mn; $YF_3$:Yb,Er; $YF_3$:Ln (Ln = Lanthanide); $YLiF_4$:Ln (Ln = Lanthanide); $Y_3Al_5O_{12}$:Ln (Ln = Lanthanide); $YAl_3(BO_4)_3$:Nd,Yb; $(Y,Ga)BO_3$:Eu; $(Y,Gd)BO_3$:Eu; $Y_2Al_3Ga_2O_{12}$:Tb; $Y_2SiO_5$:Ln (Ln = Lanthanide); $Y_2O_3$:Ln (Ln = Lanthanide); $Y_2O_2S$:Ln (Ln = Lanthanide); $YVO_4$:A (A = Lanthanide, In); $Y(P,V)O_4$:Eu; $YTaO_4$:Nb; $YAlO_3$:A (A = Pr, Tm, Er, Ce); YOCl:Yb,Er; $LnlPO_4$:Ln2 (Lnl, Ln2 = Lanthanide oder Mischungen von Lanthaniden); $A_x(PO_4)_y$:Ln (A = Erdalkali, Ln = Lanthanide) $LuVO_4$:Eu; $GdVO_4$:Eu; $Gd_2O_2S$:Tb; $GdMgB_5O_{10}$:Ce,Tb; LaOBr:Tb; $La_2O_2S$:Tb; $LaF_3$:Nd,Ce; $BaYb_2F_8$:Eu; $NaYF_4$:Yb,Er; $NaGdF_4$:Yb,Er; $NaLaF_4$:Yb,Er; $LaF_3$:Yb,Er,Tm; $BaYF_5$:Yb,Er; $Ga_2O_3$:Dy; GaN:A (A = Pr, Eu, Er, Tm); $Bi_4Ge_3O_{12}$; $LiNbO_3$:Nd,Yb; $LiNbO_3$:Er; $LiCaAlF_6$:Ce; $LiSrAlF_6$:Ce; $LiLuF_4$:A (A = Pr, Tm, Er, Ce); $Li_2B_4O_7$:Mn, $SiO_x$:Er,Al ($0 \leq x \leq 2$).

Besonders bevorzugt werden folgende Materialien als lad-Nanoteilchen verwendet: $YVO_4$:Eu, $YVO_4$:Sm, $YVO_4$:Dy, $LaPO_4$:Eu, $LaPO_4$:Ce, $LaPO_4$:Tb, $LaPO_4$:Ce,Tb, $LaPO_4$:Ce,Sm, $LaPO_4$:Ce,Dy, $LaPO_4$:Ce,Nd, ZnS:Tb, ZnS:$TbF_3$, ZnS:Eu, ZnS:$EuF_3$, $Y_2O_3$:Eu, $Y_2O_2S$:Eu, $Y_2SiO_5$:Eu, $SiO_2$:Dy, $SiO_2$:Al, $Y_2O_3$:Tb, CdS:Mn, ZnS:Tb, ZnS:Ag, ZnS:Cu. Unter den besonders bevorzugten Materialien werden insbesondere diejenigen ausgewählt, die eine kubische Gitterstruktur des Wirtsgitters besitzen, da bei diesen Materialien die Zahl der einzelnen Fluoreszenzbanden minimal wird. Beispiele hierfür sind: $MgF_2$:Mn; ZnS:Mn, ZnS:Ag, ZnS:Cu, $CaSiO_3$:Ln, CaS:Ln, CaO:Ln, ZnS:Ln, $Y_2O_3$:Ln, oder $MgF_2$:Ln (Ln = Lanthaniden).

Die Oberfläche der erfindungsgemäß eingesetzten lad-Nanoteilchen ist so präpariert, dass Affinitätsmoleküle an diese präparierte Oberfläche ankoppeln können. Das Affinitätsmolekül tritt in dem erfindungsgemäßen Assay in Wechselwirkung mit Zielmolekülen. Beispiele für Affinitätsmoleküle sind z.B. Proteine, Peptide, Oligonukleotide oder andere Nukleinsäuremoleküle oder nukleinsäureähnliche Moleküle, wie PNAs oder Morpholinos, Oligo- oder Polysaccharide, Haptene, wie Biotin oder Digoxin oder niedermolekulare synthetische oder natürliche Antigene oder Epitope.

Die Präparation der Oberfläche der lad-Nanoteilchen kann darin bestehen, dass die Oberfläche der lad-Nanoteilchen chemisch modifiziert ist und/oder reaktive Gruppen und/oder kovalent oder nicht-kovalent gebundene Verbindungsmoleküle aufweist. Auch die in die Oberfläche der lad-Nanoteilchen angebundenen Verbindungsmoleküle können reaktive Gruppen aufweisen.

Solche reaktiven Gruppen, die sowohl geladen, ungeladen oder mit Partialladungen versehen sein können, können sowohl an der Oberfläche der lad-Nanoteilchen als auch Teil der Verbindungsmoleküle sein. Mögliche reaktive funktionale Gruppen können Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbonylverbindungen, Phosphonsäuren, Phosphorsäureester, Sulfonsäuren, Azolide oder Derivate der genannten Gruppen sein.

Als Beispiele für Verbindungsmolekül seien hier Phosphonsäurederivate, Ethylenglykol, primäre Alkohole, Aminderivate, Polymere oder Copolymere, polymerisierbare Kopplungsagenzien, Silica-Hüllen und kettenförmige Moleküle mit einer der Oberfläche der lad-Nanoteilchen entgegengesetzten Polarität genannt.

Auch Nukleinsäuremoleküle können als Verbindungsmoleküle dienen. Sie bilden die Verbindung zu einem Affinitätsmolekül, das seinerseits Nukleinsäuremoleküle mit zu den Verbindungsmolekülen komplementären Sequenzen enthält.

Die Anbindung eines Affinitätsmoleküls an Verbindungsmoleküle kann kovalent oder nicht-kovalent mit Standardmethoden der Organischen Chemie erfolgen wie Oxidation, Halogenierung, Alkylierung, Acylierung, Addition, Substitution oder Amidierung des adsorbierten oder adsorbierbaren Materials. Diese Methoden zur Anbindung eines Affinitätsmoleküls an das kovalent oder nicht-kovalent gebundene Verbindungsmolekül können vor Adsorption des Verbindungsmolekül an das lad-Nanoteilchen angewendet werden, oder nachdem es bereits an das lad-Nanoteilchen adsorbiert ist. An entsprechend behandelte lad-Nanoteilchen (z.B. mit Trimethylsilylbromid), die durch die Behandlung eine veränderte (z.B. stärker geladen, polare) Oberfläche besitzen, können auch direkt Affinitätsmoleküle durch Inkubation angebunden werden.

Die Molekülgruppen A und B, die mit Donor bzw. Akzeptor markiert sind, können Bestandteil ein und desselben Moleküls sein und z.B. an dasselbe Affinitätsmolekül ankoppeln. Eine räumliche Abstandsänderung der beiden Molekülgruppen kann durch eine Konformationsänderung oder durch eine Spaltung des Moleküls hervorgerufen werden. Diese Konformationsänderung oder Spaltung des Moleküls kann Resultat einer Interaktion des gemeinsamen Affinitätsmoleküls mit einem Zielmolekül sein.

Die Molekülgruppen A und B können sich ebenso auf unterschiedlichen Molekülen befinden, wobei die Molekülgruppen A und B jeweils an eigene Affinitätsmoleküle angekoppelt sind.

Eine räumliche Abstandsänderung kann durch eine Interaktion der den Molekülgruppen A und B zugeordneten Affinitätsmoleküle mit einem gemeinsamen Zielmolekül oder miteinander hervorgerufen werden. Eine solche Interaktion kann beispielhaft eine Wechselwirkung zwischen Proteinen, z.B. eine Immunreaktion von Antigen und Antikörper, eine Hybridisierung von Nukleinsäuren oder die Interaktion zwischen Nukleinsäuren und Proteinen umfassen.

Das erfindungsgemäße Assay kann z.B. ein homogener Immunoassay zum Nachweis eines Analyten (monoklonaler oder polyklonaler Antikörper, Protein, Peptid, Oligonukleotid, Nukleinsäure, Oligo- oder Polysaccharid, Hapten oder niedermolekulares synthetisches oder natürliches Antigen) in einer Körperprobe (wie z.B. Abstriche, Sputum, Organpunktate, Biopsien, Sekrete, Liquor, Galle, Blut, Lymphflüssigkeit, Urin, Stuhl) sein. Bei homogenen Assays wird kein Wasch- oder Separierungsschritte benötigt.

Das erfindungsgemäße Assay kann auch ein heterogener Assay sein.

Das erfindungsgemäße Assay kann sowohl in Lösung als auch in festphasengestützten bzw. array-basierenden Systemen, bei denen Oligo- bzw. Polynukleotid-Stränge oder Antikörper bzw. Antigene auf einer Oberfläche immobilisiert sind, eingesetzt werden.

Für das erfindungsgemäße Assay gibt es verschiedene Gruppen von Anwendungen.

Für eine Gruppe von Anwendungen befinden sich die (F)RET-Partnern auf demselben Molekül, das bedeutet, beide (F)RET Partner sind über entsprechende Verbindungsmoleküle mit demselben Affinitätsmolekül verbunden (Anwendungsbeispiele 1a, 1b, 2, 5, 6). Durch die Bindung eines Zielmoleküls an das Affinitätsmolekül wird eine Konformationsänderung des Affinitätsmoleküls induziert, was zu einer räumlichen Änderung der Marker zueinander führt und damit einen messbaren Unterschied des (F)RET zwischen ihnen bewirkt.

Für andere Anwendungen befinden sich die (F)RET Partner auf getrennten Molekülen und sind jeweils mit einem eigenen Affinitätsmolekül gekoppelt (Anwendungsbeispiele 3, 4). Die jeweiligen Affinitätsmoleküle können so gewählt werden, dass es zu einer Interaktion von Donor und Akzeptor kommt, welche durch die Reaktion mit dem Zielmolekül hergestellt oder aufgehoben wird und dadurch eine Veränderung des Energietransfers induziert.

Anwendungsbeispiel 1 bezieht sich auf die Wechselwirkung in einem homogenen Kinase Assay mit (F)RET-Partnern auf demselben Molekül. Ein lad-Nanoteilchen und ein Chromophor sind durch eine Peptidsequenz verbunden. Die Peptidsequenz enthält eine kinasespezifische Erkennungssequenz. Wird die Peptidsequenz an dieser Stelle durch die Kinase phosphoryliert, verändert die Präsenz des Phosphates die Konformation der Peptidesequenz und dadurch messbar die Wechselwirkung zwischen den (F)RET Partnern lad-Nanoteilchen und dem Chromophor.

Anwendungsbeispiel 1b bezieht sich auf einen homogenen Immunoassay mit (F)RET-Partnern auf einem Molekül, mit dem Protein-Protein Wechselwirkungen nachgewiesen werden, wobei Antigen-Antikörper Reaktionen nur ein Beispiel dafür darstellen. Ein lad-Nanoteilchen und ein Chromophor sind durch eine Peptidsequenz verbunden. Die Peptidsequenz enthält ein Epitop . Bindet an das Epitop ein dieses Epitop spezifisch erkennender Antikörper, so verändert dies die Konformation der Peptidsequenz und dadurch messbar die Wechselwirkung zwischen den (F)RET Partnern lad-Nanoteilchen und ein Chromophor.

Das nachzuweisende Molekül kann direkt an das Affinitätsmolekül binden. Es kann jedoch auch indirekt für eine Bindung eines Moleküls an das Affinitätsmolekül verantwortlich sein. Ein Beispiel dafür wäre die Messung von $Ca^{2+}$-Konzentrationen in lebenden Zellen. Dazu wird die Calcium abhängige Bindung des Calmodulins an die Myosinleichte-Kette-Kinase (NILCK) in glatter Muskulatur ausgenutzt. Die Calmodulin-Bindungsdomäne des MLCK wirkt als Affinitätsmolekül und ist mit den (F)RET Partnern verbunden. In Abhängigkeit der $Ca^{2+}$-Konzentration bindet Calmodulin an die Bindungsdomäne und bewirkt eine Konformationsänderung der Nachweissonde und führt zu einer Veränderung des messbaren (F)RETs.

Anwendungsbeispiel 2 bezieht sich auf einen kompetitiven Immunoassay mit (F)RET-Partnern auf einem Molekül mit dem die Konzentration eines Analyten in einer Körperprobe nachgewiesen wird. Ein lad-Nanoteilchen und ein

Chromophor werden durch ein das Epitop enthaltendes Verbindungsmolekül verbunden. Das Epitop ist einem Epitop des nachzuweisenden Analyten nachempfunden. Ein Affinitätsmolekül bindet spezifisch an das Epitop. Durch Zugabe einer Probe (z.B. Körperprobe), die den nachzuweisenden Analyten enthält, wird das an das Epitop gebundene Affinitätsmolekül von dem Epitop verdrängt, was eine Konformationsänderung des Moleküls und dadurch eine messbare Änderung der Wechselwirkung zwischen den (F)RET Partnern lad-Nanoteilchen und dem Chromophor zur Folge hat. Diese Änderung des (F)RET wird zur Konzentrationsbestimmung des Analyten herangezogen.

Anwendungsbeispiel 3 bezieht sich auf einen homogenen Sättigungs-Immunoassay mit (F)RET-Partnern auf getrennten Molekülen. Die Affinitätsmoleküle des lad-Nanoteilchens und des Chromophors können unterschiedliche Epitope des gleichen Zielmoleküls erkennen, so dass es bei Anwesenheit des Zielmoleküls zu einem messbaren Energietransfer kommt

Beispielhaft für einen homogenen Immunoassay, bei dem sich Donor und Akzeptor auf getrennten Molekülen befinden, sei hier der Nachweis von hCG (humanes chorionales Gonadotropin) im Serum genannt. Dabei werden Donor und Akzeptor mit Antikörpern gekoppelt, welche unterschiedliche Epitope des hCG erkennen. Ist in einer Körperprobe hCG vorhanden, binden sowohl Donor als auch Akzeptor-Sonden an den Analyten. Der messbare FRET kann anhand einer Eichkurve in eine Konzentration des Analyten in der Körperprobe übersetzt werden.

Anwendungsbeispiel 4 bezieht sich auf einen homogenen, kompetitiven Immunoassay mit (F)RET-Partnern und auf getrennten Molekülen. Ein oder mehrere Chromophore sind mit einem Molekül verbunden, welches Teilen oder komplett dem nachzuweisenden Molekül entspricht. Ein lad-Nanoteilchen ist an ein Affinitätsmolekül gekoppelt, das spezifisch sowohl mit dem Molekül als auch mit dem nachzuweisende Molekül interagiert. Es kommt zu einer Bindung zwischen den Molekülen und und dadurch zu einem (F)RET. Wird nun eine Probe (z.B. Körperprobe) mit dem zu messenden Molekül dazugegeben, erfolgt eine Verdrängungsreaktion abhängig von der Konzentration des nachzuweisenden Moleküls in der besagten Probe. Diese führt zu einer messbaren Veränderung, in diesem Fall zu einer Verringerung des (F)RET und es kann anhand einer Eichkurve die Konzentration des nachzuweisenden Moleküls ermittelt werden.

Anwendungsbeispiel 5 bezieht sich auf einen homogenen Assay mit (F)RET-Partnern auf einem Molekül. Ein lad-Nanoteilchen und ein Chromophor werden durch ein Peptid als Affinitätsmolekül verbunden. Dieses Peptid kann durch ein nachzuweisendes Enzym gespalten werden. Nach Spaltung kommt kein (F)RET mehr zustande.

Ein Assay gemäß Anwendungsbeispiel 5 kann zum Nachweis einer bestimmten Enzymaktivität, z.B. einer für das HI-Virus spezifischen Protease innerhalb einer Probe oder Zelle verwendet werden, wobei die beiden (F)RET Partner durch die kurze Erkennungssequenz dieser Protease verbunden sind und die durch die Aktivität der Protease, nämlich der Spaltung des Peptides, räumlich voneinander getrennt werden. Die nachzuweisende Enzymaktivität kann auch eine Restriktionsendonuklease sein. Dabei sind die beiden (F)RET Partner durch eine Nukleinsäure verbunden.

Anwendungsbeispiel 6 bezieht sich auf einen Assay nach der Methode der Molecular Beacon. Molecular Beacons sind DNA-Moleküle, die sich durch intramolekulare komplementäre Sequenzen zu einer sogenannten Stem-Loop oder Haarnadelstruktur falten können. An einem Ende der DNA-Sequenz ist ein lad- Nanoteilchen angekoppelt, an dem anderen Ende befindet sich ein Chromophor als Fluoreszenzlöscher oder Quencher. In der Haarnadelstruktur sind die beiden (F)RET Partner und eng benachbart angeordnet, die Fluoreszenz des Donors daher vollständig gelöscht. Das nachzuweisende Zielmolekül weist Sequenzen auf, die komplementär zu der Schlaufen Region der DNA-Sequenz sind. Da die Bindung an das Zielmolekül energetisch günstiger ist, wird die Haarnadel Konformation aufgelöst, Chromophor und lad-Nanoteilchen lösen sich voneinander und Fluoreszenz wird messbar emittiert, da es nicht mehr zu einer Fluoreszenzlöschung durch (F)RET kommt. Die Hybridisierungseigenschaften können so eingestellt werden, dass bereits eine einzelne Fehlbasenpaarung zwischen dem Molecular Beacon und der Ziel-DNA nicht zur Öffnung der Haarnadelstruktur führt. Dadurch lassen sich sogar einzelne Basenunterschiede (z.B. SNPs, single nucleotide polymorphisms) detektieren.

**Beispiele für Verbindung von lad-Nanoteilchen mit organischen Molekülen**

**Beispiel 1: Anbindung von Phosphonsäure-Derivaten**

Die chemischen Modifikation der Oberfläche des lad-Nanoteilchens kann z.B. über die Anbindung von Phosphonsäure-Derivaten mit funktionalen reaktiven Gruppen erfolgen. Dabei werden an die lad-Nanoteilchen Phosphonsäure- oder Phosphonsäureester-Derivate, wie z.B. Imino-bis(Methylenphosphono)Carbonsäure (kann beispielsweise hergestellt werden mittels der Mannich-Moedritzer Reaktion, Moedritzer und Irani, J. Org. Chem., 1966, 31, 1603), stabil an die Oberfläche angebunden. Diese Anbindung kann auf unbehandelten oder auf zuvor behandelten (z.B. mit Trimethylsilylbromid) lad-Nanoteilchen, stattfinden. Mögliche reaktive funktionale Gruppen, die diese Phosphonsäure- oder Phosphonsäureester enthaltenden Verbindungsmoleküle tragen, können Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbonylverbindungen, Phosphonsäuren, Phosphorsäureester, Sulfonsäuren, Azolide oder Derivate der genannten Gruppen sein.

**Beispiel 2: Anbindung von Ethylenglykol, primären Alkoholen, primären Aminderivaten**

Ein weiteres Beispiel für die Oberflächenbehandlung der lad-Nanoteilchen ist das Erhitzen der Partikel in Ethylenglykol, wobei unter Abspaltung der Alklyketten auf der lad-Nanoteilchen eine stabile Anbindung des Ethylenglykols erfolgt. Diese Behandlung resultiert in einer Wasserlöslichkeit der Partikel. In analoger Weise können primäre Alkohole mit funktionalen reaktiven Gruppen, wie oben angegeben, verwendet werden. In ähnlicher Weise kann an die Oberfläche der lad-Nanoteilchen ebenfalls primäre Aminderivate stabil angebunden werden. Die Aminderivate können zusätzlich zu der Amingruppe die oben angegebenen reaktiven funktionalen Gruppen enthalten.

**Beispiel 3: Umhüllung mit Silica, Polymeren oder Copolymeren und polymerisierbaren Kopplungsagenzien**

Als weiteres Beispiel einer chemischen Modifikation der Oberfläche des lad-Nanoteilchens sei die Umhüllung eines lad-Nanoteilchens oder einer Gruppe von lad-Nanoteilchen mit Silica genannt: Silica ermöglicht eine einfache chemische Konjugation von organischen Molekülen, da Silica sehr leicht mit organischen Linkern wie z.B. Triethoxysilanen oder Chlorsilanen reagiert. Ein weiteres Beispiel einer chemischen Modifizierung ist die Umhüllung eines lad-Nanoteilchens oder einer Gruppe von lad-Nanoteilchen mit Polymeren oder Copolymeren. Als Beispiel für polymerisierbare Kopplungsagenzien seien N-(3-aminopropyl)3-mercaptobenzamidine, 3-(trimethoxysilyl)propylhydrazid und 3-(trimethoxysilyl) propylmaleimid genannt. Ebenso können eine Vielzahl von verschiedenen polymerisierbaren Kopplungsagenzien zusammen verwendet werden, um ein oder mehrere lad-Nanoteilchen mit einer umhüllenden Schicht zu versehen. Vor allem bei Kopplungsagenzien, die nur eine schwache Bindung zum lad-Nanoteilchen aufweisen, kann das von Vorteil sein. Beispiele für Kopplungsagenzien, die solche Netze als Umhüllung eines oder mehrerer lad-Nanoteilchen ausbilden können, sind Diacetylene, Styrolbutadiene, Vinylacetat, Acrylate, Acrylamide, Vinyle, Styrole, Silikonoxide, Boroxide, Phosphoroxide, Borate, Pyrrole, Polypyrrole und Phosphate sowie Polymere von zumindest einigen der besagten Agenzien.

**Beispiel 4: Oberflächenmodifikation mit Oxichloriden, sowie nicht-kovalente Verknüpfungen mit kettenförmigen Molekülen und amphiphilen Reagenzien**

Eine weitere Möglichkeit der Präparation der Oberfläche der lad-Nanoteilchen besteht darin, die oxidischen Übergangsmetallverbindungen, aus denen die lad-Nanoteilchen bestehen, mit Chlorgas oder organischen Chlorierungsagenzien in die entsprechenden Oxichloride zu überführen. Diese Oxichloride reagieren ihrerseits mit Nukleophilen wie z.B. Aminogruppen unter Bildung von Übergangsmetallstickstoffverbindungen. Auf diese Weise lässt sich zum Beispiel über die Aminogruppen von Lysinseitenketten eine direkte Konjugation von Proteinen erzielen. Die Konjugation mit Proteinen nach der Oberflächenmodifikation mit Oxichloriden kann auch durch Verwendung eines bifunktionellen Linkers wie Maleimidopropionsäurehydrazid bewerkstelligt werden.

Für nicht-kovalente Verknüpfungen eignen sich dabei besonders kettenförmige Moleküle mit einer der Oberfläche des lad-Nanoteilchen entgegengesetzten Polarität oder Ladung. Als Beispiele für Verbindungsmoleküle, die nicht-kovalent mit den lad-Nanoteilchen verknüpft sind, seien anionische, kationische oder zwitterionische Detergenzien, saure oder basische Proteine, Polyamine, Polyamide, Polysulfon- oder Polycarbonsäuren genannt. Durch hydrophobe Wechselwirkung zwischen den lad-Nanoteilchen und amphiphilen Reagenzien, die eine funktionale reaktive Gruppe tragen, kann eine Verknüpfung hergestellt werden. Besonders Kettenmoleküle, die einen amphiphilen Charakter haben wie z.B. Phospholipide oder derivatisierte Polysaccharide, die untereinander vernetzt werden können, sind dafür geeignet Die Adsorption dieser Moleküle auf die Oberfläche des lad-Nanoteilchens lässt sich durch Koinkubation erzielen.

**Beispiel 5: Anbindung von Affinitätsmolekülen an lad-Nanoteilchen mit Verbindungsmolekül**

Generell können solche Affinitätssmoleküle verwendet werden, wie sie auch bei den im Stand der Technik beschriebenen fluoreszierenden organischen Farbstoffmolekülen benutzt werden, um diese spezifisch an die nachzuweisende biologische oder sonstige organische Substanz zu binden. Ein Affinitätssmolekül kann ein monoklonaler oder polyklonaler Antikörper, ein anderes Protein, ein Peptid, ein Oligonukleotid, ein Plasmid oder ein anderes Nukleinsäuremolekül, ein Peptid Nucleic Acid (PNA) oder ein Morpholino, ein Oligo- oder Polysaccharid oder ein Hapten, wie Biotin oder Digoxin oder ein niedermolekulares synthetisches oder natürliches Antigen sein. Eine Liste solcher Moleküle sind in der allgemein zugänglichen Literatur veröffentlicht, z. B. in "Handbook of Fluorescent Probes and Research Chemicals" (8th edition, 2001, CD-ROM) von R. P. Hauglund, Molecular Probes, Inc..

Um Affinitätsmoleküls kovalent oder nicht-kovalent an die lad-Nanoteilchen mit Verbindungsmolekül zu binden,

werden reaktive Gruppen auf der Oberfläche des Affinitätsmoleküls genutzt. Reaktive Gruppen auf der Oberfläche des Affinitätsmoleküls sind dabei Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbonylverbindungen, Phosphonsäuren, Phosphorsäureester, Sulfonsäuren, Azolide oder Derivate der genannten Gruppen. Auf der Oberfläche der lad-Nanoteilchen können die weiter oben beschriebenen funktionalen reaktiven Gruppen der Verbindungsmoleküle zur Konjugation des Affinitätsmoleküls verwendet werden. Protokolle für Kopplungen an reaktive Gruppen sind in der allgemein zugänglichen Literatur beschrieben, z.B. in "Bioconjugate Techniques" (von Greg T. Hermanson, Academic Press 1996).

Sind Donor und Akzeptor auf demselben Molekül enthalten, kann an das Affinitätsmolekül, welches über ein oder mehrere Verbindungsmoleküle an den Donor gekoppelt ist, kovalent oder nicht-kovalent an den Akzeptor angebundenen werden. Als Akzeptor können Chromophore verwendet werden, deren Anregungswellenlänge mit der Emissionswellenlänge des jeweiligen Donors überlappen. Akzeptoren können z.B. lad-Nanoteilchen, organische Farbstoffe, (z.B. Fluorescein, Rhodamin, Cyanine), organische Pigmente (z.B. Perylene) oder, leitfähige Materialien (z.B. Metalle, dotierte Oxide, leitfähige Polymere) sein. Diese Materialien können entweder als partikuläre Systeme oder als ebene oder strukturierte Oberfläche bzw. Oberflächenbeschichtung vorliegen. Die Kopplung kann über reaktive Gruppen auf dem Affinitätsmolekül und/oder dem Akzeptormolekül stattfinden.

Außer kovalenten Verknüpfungen von Affinitätsmolekül sind nicht-kovalente, selbstorganisierte Verbindungen realisierbar. Als eine Möglichkeit sei hierbei die Verknüpfung von einfachen Nachweissonden mit Biotin als Verbindungsmolekül mit Avidin- oder Streptavidin- gekoppelten Affinitätsmolekülen genannt.

**<u>Beispiel 6:</u> Die Herstellung von lad-Nanoteilchen bestehend aus $YVO_4$:Eu**

Der erste Schritt besteht in der Bereitstellung von $YVO_4$:Eu. $YVO_4$:Eu kann nach dem in K. Riwotzki, M. Haase; Journal of Physical Chemistry B; Vol. 102, 1998, Seite 10130, linke Spalte, angegebenen Verfahren hergestellt werden: In einem Teflonbehälter wird 3,413 g $Y(NO_3)_3{\cdot}6H_2O$ (8,9 mmol) und 0,209 g $Eu(NO_3)_3{\cdot}6H_2O$ (0,47 mmol) in 30 mL destilliertem Wasser gelöst. Dazu wird unter Rühren 2,73 g $Na_3(VO_4){\cdot}10H_2O$, das in 30 mL destilliertem Wasser gelöst wurde, gegeben. Nach 20 Min. weiterem Rühren wird der Teflonbehälter in einen Autoklaven gestellt und unter weiterem Rühren auf 200°C erhitzt. Nach 1h wird die Dispersion aus dem Autoklaven entnommen und bei 3000g 10 Min. zentrifugiert und derÜberstand verworfen. Das Präzipitat wird in 40 mL destilliertem Wasser aufgenommen. 3,220 g einer wässrigen 1-Hydroxyethan-1,1-Diphosphonsäure-Lösung (60 Gew.-%) wird zu der Dispersion gegeben (9,38 mmol). Zur Entfernung von $Y(OH)_3$, das sich aus überschüssigen Yttriumionen gebildet hat, wird der pH-Wert mit $HNO_3$ auf 0,3 eingestellt und 1h gerührt. Dabei bildet sich kolloidales $V_2O_5$, das sich durch eine rötliche Färbung der Lösung bemerkbar macht. Danach wird der pH-Wert mit NaOH auf 12,5 eingestellt und über Nacht in einem geschlossenen Behälter gerührt. Die resultierende weiße Dispersion wird dann bei 3000g für 10 Min.. zentrifugiert und der Überstand mit seinen Nebenprodukten entfernt. Der Niederschlag besteht aus $YVO_4$:Eu und kann mit 40 mL destilliertem Wasser aufgenommen werden.

Zur Isolation der Nanoteilchen, die kleiner als ca. 30 nm sind, wird die Dispersion bei 3000g für 10 Min.. zentrifugiert, der Überstand dekantiert und zurückgestellt. Anschließend wurde der Niederschlag noch einmal mit 40 mL destilliertem Wasser aufgenommen, bei 3000g für 10 Min.. zentrifugiert und der Überstand dekantiert. Dieser und der zurückgestellte Überstand wurden zusammen gegeben und bei 60000g 10 Min. zentrifugiert. Der hieraus resultierende Überstand enthält die gewünschten Teilchen. Nach einem weiteren Dialyseschritt gegen destilliertes Wasser (Dialyseschlauch Serva, Heidelberg, MWCO 12 - 14 kD) erhält man eine kolloidale Lösung, aus der durch Trocknung mit einem Rotationsverdampfer (50°C) ein redispergierbares Pulver gewonnen werden kann.

**<u>Beispiel 7:</u> Die Herstellung von lad-Nanoteilchen bestehend aus $LaPO_4$:$Eu^{3+}$**

Der erste Schritt besteht in der Bereitstellung von $LaPO_4$:Eu. $LaPO_4$:Eu kann nach dem in H. Meyssamy, K. Riwotzki, A. Kornowski, S. Naused, M. Haase; Advanced Materials, Vol. 11, Issue 10, 1999, Seite 843, rechte Spalte unten bis Seite 844, linke Spalte oben, angegebenen Verfahren hergestellt werden: In einem Teflonbehälter wird 12,34 g $La(NO_3)_3{\cdot}6H_2O$ (28,5 mmol) und 0,642 g $Eu(NO_3)_3{\cdot}5H_2O$ (1,5 mmol) in 50 mL destilliertem Wasser gelöst und in 100 mL NaOH (1 M) gegeben. Dazu wird unter Rühren eine Lösung aus 3,56 g $(NH_4)_2HPO_4$ (27 mmol) in 100 mL destilliertem Wasser gegeben. Die Lösung wird mit NaOH (4 M) auf pH 12,5 eingestellt und in einem Autoklaven 2 h bei 200°C unter kräftigem Rühren erhitzt. Die Dispersion wird dann bei 3150g 10 Min.. zentrifugiert und der Überstand entfernt. Um unerwünschtes $La(OH)_3$ zu entfernen, wird der Niederschlag in $HNO_3$ (1M) dispergiert und 3 Tage gerührt (pH 1). Danach wird die Dispersion zentrifugiert (3150g, 5 Min.) und der Überstand entfernt. Dem Zentrifugat wird unter Rühren 40 mL destilliertes Wasser zugesetzt.

Die milchige Dispersion enthält noch eine breite Größenverteilung. Zur Isolation der Nanoteilchen, die kleiner

als ca. 30 nm sind, werden in vollständiger Analogie zu Beispiel 6 entsprechende Zentrifugations- und Dekantierungsschritte nachgeschaltet.

**Beispiel 8: Die Herstellung von lad-Nanoteilchen bestehend aus $LaPO_4$:Ce, Tb**

Der erste Schritt besteht in der Bereitstellung von $LaPO_4$:Ce,Tb. 300 mL Trisethylhexylphosphat werden mit einem trockenen Stickstoff-Gasstrom entgast. Anschließend werden 7,43 g $LaCl_3 \cdot 7H_2O$ (20 mmol), 8,38 g $CeCl_3 \cdot 7H_2O$ (22,5 mmol) und 2,8 g $TbCl_3 \cdot 6H_2O$ (7,5 mmol) in 100 mL Methanol gelöst und hinzugegeben. Danach wird Wasser und Methanol durch Heizen der Lösung auf 30°C bis 40°C im Vakuum abdestilliert. Eine frisch hergestellte Lösung bestehend aus 4,9 g trockene Orthophosphorsäure (50 mmol), die in einer Mischung aus 65,5 mL Trioctylamin (150 mmol) und 150 mL Trisethylhexylphosphat gelöst wurden, werden anschließend hinzugegeben. Die klare Lösung muss rasch in ein zu evakuierendes Gefäß gestellt und mit Stickstoff-Gasstrom gespült werden, um die Oxidation von $Ce^{3+}$ bei einer Temperaturerhöhung zu minimieren. Anschließend wird die Lösung auf 200°C erhitzt. Während der Heizphase werden einige der Phosphorsäureester-Gruppen gespalten, was zu einer allmählichen Erniedrigung der Siedepunktes führt. Die Heizphase wird beendet, wenn die Temperatur auf 175°C fällt (ca. 30 bis 40 h). Nachdem die Lösung auf Zimmertemperatur abgekühlt ist, wird ein 4-facher Überschuß an Methanol hinzugegeben, was zu einer Ausfällung der Nanoteilchen führt. Der Niederschlag wird abgetrennt, mit Methanol gewaschen und getrocknet.

**Beispiel 9: Die Herstellung von lad-Nanoteilchen bestehend aus $LaPO_4$:$Eu^{3+}$**

490 mg (5,0 mmol) trockene Orthophosphorsäure und 6,5 mL (15 mmol) Trioctylamin werden in 30 mL Trisethylhexlphosphat gelöst. Anschließend werden 1,76 g $La(NO_3)_3 \cdot 7H_2O$ (4,75 mmol) und 92 mg $EuCl_3 \cdot 6H_2O$ (0,25 mmol) in 50 mL Trisethylhexlphosphat gelöst und mit der ersten Lösung vereint. Die resultierende Lösung wird unter Vakuum entgast und anschließend für 16 h unter Stickstoff bei 200°C geheizt. Während der Heizphase werden einige der Phosphorsäureester-Gruppen gespalten, was zu einer allmählichen Erniedrigung der Siedepunktes führt. Die Heizphase wird beendet, wenn die Temperatur auf 180°C fällt. Nachdem die Lösung auf Zimmertemperatur abgekühlt ist, wird Methanol hinzugegeben, was zu einer Ausfällung der Nanoteilchen führt. Der Niederschlag wird mit Hilfe einer Zentrifuge abgetrennt, mit Methanol zweimal gewaschen und getrocknet.

**Beispiel 10: Lösen der in Beispiel 8 dargestellten Nanopartikel in Wasser durch Umsetzung von Ethylen- oder Polyethylenglykol**

50 mg der in Beispiel 8 hergestellten $LaPO_4$:Ce,Tb Nanopartikel (~140 nmol) werden mit 5 mL Ethylenglykol (~180 mmol) (alternativ können auch andere Alkohole, insbesondere Diole, bevorzugt Polyethylenglykole verschiedener Kettenlänge, $HO-(CH_2-CH_2-O)_n-OH$, wobei n = 2 - 9, verwendet werden) und 5 µl Schwefelsäure (96-98 %) unter Rühren und Inertgas 3 Std. bei 210°C erhitzt. Die Partikel gehen bei ca. 135°C in Lösung. Anschließend wird ein Wasserstrahlvakuum von ca. 1,5 mbar angelegt und etwa die Hälfte des Ethylenglykols abdestilliert, der Rückstand bleibt klar. Anschließend wird gegen Wasser über Nacht dialysiert (Dialyseschlauch Spectra/Por, 5-6.000 MWCO, Spektrum, Niederlande).

**Beispiel 11: Carboxy-Funktionalisierung von in Beispiel 10 hergestellten Nanopartikeln durch Oxidation**

Zu 100mg (~300nmol) in 20 mL Wasser der in Beispiel 10 dargestellten Nanopartikel wird unter Rühren zunächst 0.5 mL 96-98%ige Schwefelsäure dazugegeben. Tropfenweise wird 1mM $KMnO_4$ Lösung dazugegeben. bis keine Entfärbung der violetten Farbe mehr auftritt. Man gibt anschließend nochmals dieselbe Menge $KMnO_4$- Lösung hinzu und lässt über Nacht bei Raumtemperatur nachrühren (>12 h). Überschüssiges Permanganat wird durch tropfenweise Zugabe von frisch bereiteter 1mM Natriumsulfit Lösung reduziert. Über Nacht wird gegen 0,1M MES, 0,5M NaCl, pH 6,0 dialysiert (Dialyseschlauch Spectra/Por, 5-6.000 MWCO, Spektrum, Niederlande).

**Beispiel 12: Entfernung der Alklyketten des Trisethylhexylphosphats von der Oberfläche der in Beispiel 8 beschriebenen Nanopartikeln durch Reaktion mit Bromtrimethylsilan**

300 mg der in Beispiel 8 dargestellten $LaPO_4$:Ce,Tb Nanopartikel (~ 850 nmol) werden mit 2, 3 g (15 mmol) Bromtrimethylsilan, in 100 mL Chloroform für 4 Stunden unter Rückfluss gekocht, Bromtrimethylsilan-Überschuß und entstandenes Zwischenprodukt größtenteils abdestilliert und anschließend leicht amoniakalisch hydrolysiert. Dazu werden 6 mL Wasser mit 100 µl 25%igem Amoniak versetzt und über Nacht bei RT gerührt. Die Partikel liegen in einer milchigen Emulsion vor und setzten sich nach mehreren Stunden zum Teil ab.

**Beispiel 13: Die Kopplung von in Beispiel 12 dargestellten Nanopartikeln mit 11-Bis(phosphorylmethyl)amino-undecansäure und 1,4-Bis(3-Aminopropoxy)-Butan**

Zur Herstellung von 11-Bis(phosphorylmethyl)amino-undecansäure werden 201 g 11-Aminoundecansäure, 170 g Phosphorige Säure, 200ml konzentriere Salzsäure und 200ml Wasser vorgelegt und auf 100°C erhitzt. Dann werden innerhalb von 1h 324 g Formalin (37%) zugetropft und noch 1h bei 100°C nachgerührt. Nach Abkühlen auf Raumtemperatur wird das Produkt abgesaugt und im Vakuum getrocknet. Man erhält 334g 11-Bis(phosphorylmethyl) amino-undecansäure, die durch Elementaranalyse (C,H,N,P) charakterisiert wurde.

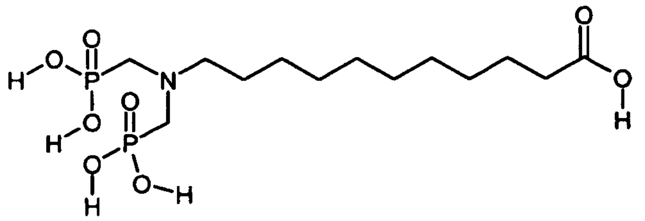

Es können alternativ Moleküle folgender Formel eingesetzt werden, wobei der Rest (R) ein Alkylen mit 1 bis 17 Kohlenstoffatomen, vorzugsweise 3-12 Kohlenstoffatomen, oder eine Alkylenarylenrest mit 7-12 Kohlenstoffatomen ist:

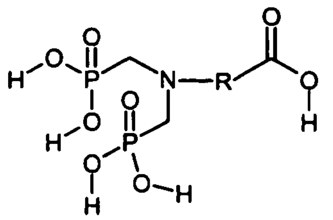

0,5 g (1,85 mmol) 11-Bis(phosphorylmethyl)amino-undecansäure werden in 2 mL Ethylenglykol mit 0,894 g (4,375 mmol) 1,4-Bis(3-Aminopropoxy)-Butan versetzt. Nachdem sich eine klare Lösung gebildet hat (exotherme Reaktion), werden 35 mg (= 100 nmol) der in Beispiel 12 dargestellten Nanopartikel bei ca. 50°C hinzugefügt und der Ansatz auf 125°C erhitzt. Die Partikel gehen bei 120°C vollständig in Lösung. Nach 4 Std. liegt eine klare hellbraune Lösung vor, die auch nach Abkühlung auf RT klar bleibt. Über Nacht wird gegen 2x2 L 10mM Na-Carbonatpuffer pH 8,5 dialysiert (Dialyseschlauch Spectra/Por, 5-6.000 MWCO, Spektrum, Niederlande). Das Dialysat ist klar.

**Beispiel 14: Biotinylierung von in Beispiel 13 dargestellten Nanoteilchen**

6,2 mL der in Beispiel 13 dargestellten Partikel (= 5 mg oder ~15 nmol) werden am Rotavapor eingeengt und auf 4,81 mg/mL aufkonzentriert. Die Partikel werden mit einem 20-fach molaren Überschuss von in Wasser gelöstem Biotin-X-NHS (Sulfo-Biotin-Aminocapronsäure-N-Hydroxy-Succinimid-Ester, Calbiochem, Schwalbach) für 4 Std. bei RT rotierend inkubiert und anschließend gegen PBS-Puffer (8 mM $K_2HPO_4$; 150 mM NaCl; 2 mM $Na_2HPO_4$; pH 7,4) dialysiert (Dialyseschlauch Spectra/Por, 5-6.000 MWCO, Spektrum, Niederlande). Das Dialysat ist leicht trüb.

**Beispiel 15: Kopplung eines DNA Oligonukleotids an die in Beispiel 13 dargestellten Nanopartikel**

Die in Beispiel 13 dargestellten Nanopartikel werden mit einem 40-fachen Überschuss von sulfo-SIAB (Sulfosuccinimidyl(4-iodoacetyl)aminobenzoat, Perbio Science Deutschland GmbH, Bonn) aktiviert: 7,5 mg aminofunktionalisierte Nanopartikel (~ 25 nmol) werden mittels einer Centricon Filtereinheit (MW-Ausschlussgrenze bei 50 000, Millipore, Eschborn) in TSMZ-Puffer pH 7,3 (0,1 M NaCl; 0,1 M Triethanolamin-HCl; 0,02 M NaOH; 0,27 mM $ZnCl_2$; 0,1% Tween 20; 1mM $MgCl_2$) umgepuffert und auf eine Konzentration von etwa 7 mg/mL eingestellt. Zu der Partikel Lösung werden 50 μl einer 20 mM sulfo-SIAB Lösung in Wasser gegeben und für 15 Min. bei 25°C inkubiert. Die Reaktion wird durch Zugabe von 12 μl 1 M Glycin (12-facher Überschuss) gestoppt und das freie sulfo-SIAB über eine Sephadex G25 PD 10 Fertigsäule (Amersham Pharmacia Biotech, Freiburg) abgetrennt. Ein DNA Oligonukleotid der Sequenz 5'-CCACGCTTGTGGGTCAACCCCCGTGG-3' mit einer Thiol-Modifikation am 5'-Ende und einer Dabycl-Modifikation

(4-(4-Dimethylminophenylazo) benzoyl) am 3'-Ende, sowie ein Kontroll DNA-Oligonukleotid, welches sich lediglich durch das Fehlen des Dabcyl-Moleküls am 3'-Ende von der Sonde unterscheidet, wurden bei Interactiva (Ulm) bestellt. Es werden äquimolare Mengen des DNA Oligonukleotids und der SIAB aktivierten Nanopartikel vermischt und 3 Std. bei 25°C und anschließend über Nacht bei 4°C inkubiert. Die DNA Oligonukleotid gekoppelten Partikel werden von nicht gekoppelten Partikeln und freiem DNA Oligonukleotid über eine FPLC (Fast Performance Liquid Chromatography) abgetrennt. Die gekoppelten Partikel werden in 50 mM Tris-HCl, pH 7,4; 0,1% BSA bei 4°C aufgehoben. Ist keine Ziel-DNA vorhanden, liegt dieses Moleküle in einer hairpin Struktur vor, wobei die Enden des Moleküls direkt benachbart sind und ein FRET stattfindet. Die Fluoreszenz des Nanoteilchens wird dabei durch Dabcyl gelöscht.

**Beispiel 16: Kopplung von anti-β-hCG monoklonalem Antikörper an die in Beispiel 13 dargestellten Nanopartikel**

Zunächst werden die in Beispiel 13 dargestellten Partikel mit einem 30-fachen molaren Überschuss von 2-Iminothiolan (2-IT, Traut's reagent, Perbio Science Deutschland GmbH, Bonn) aktiviert: 2 mL (~25 nmol) der in Beispiel 13 dargestellten Partikel (4mg/mL) werden in TSE-Puffer pH 8,5 (0,04 M NaCl; 0,05 M Triethanolamin-HCl; 0,04 M NaOH; 0,5 mM EDTA; 0,1% Tween 20; pH 8,5) überführt. Dazu werden die Partikel 3x 15 Min. bei 3000g zentrifugiert und der Niederschlag nach Abdekantieren des Überschusses jeweils in 700 µl TSE-Puffer pH 8,5 aufgenommen. Diese Partikel werden mit 75 µl 10mM 2-IT (in TSE-Puffer pH 8,5) 1 Std. bei 25°C inkubiert und die Reaktion anschließend mit 9 µl (12-fachem Überschuss) 1 M Glycin abgestoppt. Zum Abtrennen des 2-IT Überschusses wird erneut 3x 15 Min. bei 3000g zentrifugiert und der Niederschlag nach Abdekantieren zweimal in 1 mL TSE-Puffer pH 7,3 (0,1 M NaCl; 0,1 M Triethanolamin-HCl; 0,02 M NaOH; 1mM EDTA; 0,1% Tween 20; pH 7,3), nach der dritten Zentrifugation in 250 µl TSE-Puffer pH 7,3 resuspendiert. Gleichzeitig wird eine äquimolare Menge des monoklonalen Maus Antikörpers spezifisch für β-hCG (Klon F199C1, Perkin-Elmer Life Sciences - Wallac Oy, Finnland) mit einem 40-fachen Überschuss von SMCC (N-Succinimidyl-4-(N-Maleimido-methyl)-cyclohexan-1-carboxylat, Perbio Science Deutschland GmbH, Bonn) aktiviert: 750 µL anti β-hCG Antikörper (= 25 nmol bei einer Konzentration von 5 mg/mL) werden mittels einer Centricon Filtereinheit (Molekulargewichts-ausschlussgrenze bei 50 000) in TSMZ-Puffer pH 7,3 (0,1 M NaCl; 0,1 M Triethanolamin-HCl; 0,02 M NaOH; 0,27 mM $ZnCl_2$; 0,1% Tween 20; 1mM $MgCl_2$) umgepuffert und auf eine Konzentration von 7 mg/mL eingestellt. Zu der Antikörper Lösung werden 50 µl einer 20 mM SMCC-Lösung in DMF (= 1mmol) gegeben und für 30 Min. bei 25°C inkubiert. Die Reaktion wird durch Zugabe von 12µl 1 M Glycin (12-facher Überschuss) gestoppt und das freie SMCC über eine Sephadex G25 PD 10 Fertigsäule (Amersham Pharmacia Biotech, Freiburg) abgetrennt. Schließlich werden äquimolare Mengen der 2-IT aktivierten Partikellösung und der SMCC aktivierten Antikörperlösung vermischt und 3 Std. bei 25°C und anschließend über Nacht bei 4°C inkubiert. Die Antikörper gekoppelten Partikel werden von nicht gekoppelten Partikeln und freiem Antikörper durch Gelpermeationschromatographie auf Superdex 200 (Amersham Pharmacia Biotech, Freiburg) gereinigt. Als Laufpuffer wird 0,1M MES, 0,5M NaCl, pH 6,0 verwendet. Die Retentionszeit für die erweiterten lad-Nanopartikel beträgt etwa 2 Stunden.

**Beispiel 17: Kopplung der in Beispiel 9 dargestellten LaPO4: $Eu^{3+}$ Nanoteilchen mit hIL-2:**

300 mg der in Beispiel 9 dargestellten LaP04: $Eu^{3+}$ Nanopartikel (~1 µmol) werden mit 2,23 g (15 mmol) Bromtrimethylsilan in 125 mL Chloroform für 4 Stunden unter Rückfluss gekocht, Bromtrimethylsilan-Überschuss und entstandenes Zwischenprodukt größtenteils abdestilliert und anschließend leicht amoniakalisch hydrolysiert. Dazu werden 6 mL Wasser mit 100 µl 25%igem Amoniak versetzt und über Nacht bei RT gerührt. Die Partikel liegen in einer milchigen Emulsion vor und setzten sich nach mehreren Stunden zum Teil ab. 5 mg (= 25 mmol, 106 µl) dieser Bromtrimethylsilan behandelten Nanopartikel werden mit rekombinantem humanen IL-2 Protein (R&D Systems, Mineapolis, MN, USA) in 10 mM Na-Carbonat-Puffer pH 8,5 in einem molaren Verhältnis 2:1 für 1 Std. bei 37°C schüttelnd inkubiert. Anschließend wird das überschüssige Protein durch 6-maliges Zentrifugieren für 10 Min. bei 3000g. und jeweiligem Resuspendieren in 1 mL 10mM Na-Carbonatpuffer pH 8,5 abgetrennt.. Das $LaPO_4$: $Eu^{3+}$/IL-2 Konjugat wird bei 4°C gelagert.

**Beispiel 18: Homogener Energie Transfer Assay zur Detektion von β-hCG mit in Beispiel 16 dargestellten Antikörper-gekoppelten Nanoartikeln und Fluorescein-gekoppelten Antikörpern als Akzeptor**

Kopplung von anti-β-hCG Antikörper mit Fluorescein:

Zur Kopplung des anti-β-hCG Antikörpers (M15294, Perkin-Elmer Life Sciences - Wallac Oy, Finnland) mit Fluorescein wird der Fluoreporter® FITC Protein Labeling Kit von Molecular Probes nach Angaben des Herstellers verwendet. 0,5 mg des Antikörpers werden mit Hilfe einer Centricon Filtereinheit (Molekulargewichtsausschlussgrenze bei 50 000) in 0,2 M Hydrogencarbonat Puffer pH 9,0 umgepuffert. Die Antikörperlösung wird dann mit einem 25-fachem Überschuss einer 5 mM Fluoresceinisothiocyanat (FITC) Lösung (gelöst in einem Gemisch gleicher Volumeneinheiten

DMF und 0,2 M Hydrogencarbonat Puffer pH 9,0) versetzt und 3 Std. bei RT inkubiert. Der FITC-Überschuss wird über eine Sephadex G25 PD 10 Fertigsäule (Amersham Pharmacia Biotech, Freiburg) abgetrennt, die Antikörperkonzentration und die Ratio Fluorescein/Antikörper spektroskopisch bestimmt. Das Konjugat wird mit 0,01% Natriumazid und 0,1% BSA versetzt und bei 4°C aufbewahrt.

Durchführung des Assays:

50 μl eines β-hCG Standards aus einem kommerziell erhältlichen Kit für die Messung von freiem β-hCG in Serum (A007-101, Perkin-Elmer Life Sciences - Wallac Oy, Finnland) werden zusammen mit 100 nmol des in Beispiel 16 dargestellten Nanopartikel-Antikörper Konjugates und 100 nmol des Fluorescein gekoppelten anti-β-hCG Antikörpers in 200 μl Tris-HCl Puffer, pH 7,4 in einer UVdurchlässigen 96-well Microtiterplatte (UVStar, Greiner) für 60 Min. bei 25°C inkubiert. Die beiden anti-β-hCG Antikörper sind gegen unterschiedliche Epitope der β-hCG Untereinheit gerichtet. Die Proben werden anschließend in einem Fluoreszenz-Spektrometer (Fa. Jobin Yvon, Fluorolog 3) mit folgenden Einstellungen gemessen: Gepulste Anregung mit Anregungswellenlänge: 280 nm, Emission: 542 nm, Spaltbreite: 4 nm, Zeitverzögerung (time delay): 50 μs, Repetitionsrate ca. 25 Hz vermessen. Ebenso kann die Halbwertszeit der Terbium-Emissionslinie bestimmt werden. Dazu werden folgende Einstellungen verwendet: Anregung: 280 nm, Emission 542 nm, Spaltbreite 5nm; Integrationszeit: 0,1 ms. Diese erhaltenen Messungen werden gegen die eingesetzten β-hCG Konzentrationen zur Erstellung einer Eichkurve aufgetragen. Der β-hCG Gehalt von Körperprobe kann in analoger Weise in Serum Proben gemessen werden und anhand der Eichkurve die Konzentration bestimmt werden.

**Beispiel 19: Homogener kompetetiver Energie Transfer Assay zur Bestimmung von hIL-2 mit in Beispiel 17 dargestellten Nanoteilchen gekoppelt mit hIL-2 und Alexa Fluor 680-gekoppelten anti-hIL-2Rα-chain Antikörpern**

Kopplung des monoklonalen anti-hIL-2Rα-chain Antikörper mit Alexa Fluor 680:

1 mg des monoklonalen Antikörpers 7G7B6, der die α-Kette des humanen Interleukin-2 Rezeptors (hIL-2Rα-chain) spezifisch erkennt (ATCC, Rockville, USA), wird gegen PBS dialysiert, auf eine Konzentration von 2 mg/mL eingestellt und mit dem Alexa Fluor 680 Protein Labeling Kit (Molecular Probes Europe BV, Niederlande) nach Angaben des Herstellers markiert. Als Reaktionspuffer wird 0,1 M Na-Bicarbonat Puffer pH 8,3 verwendet und für 1 Std. bei RT inkubiert. Der gekoppelte Antikörper wird über eine im Kit enthaltene Säule aufgereinigt, als Elutionspuffer wird PBS-Puffer mit 0,2 mM Na-Azid verwendet. Für die Bestimmung der Proteinkonzentration des gekoppelten Antikörpers wird die Absorption (A) bei 280 und 679 nm in einer 1 cm Küvette gemessen und nach folgender Formel berechnet:

$$M = \frac{(A_{280}-(A_{679} \times 0{,}05)) \times \text{Verdünnungsfaktor}}{203\,000}$$

wobei 203 000 $cm^{-1}M^{-1}$ der molare Extinktionskoeffizient eines IgG ist und 0,05 der Faktor zur Korrektur der Absorption des Farbstoffes bei 280 nm. Die Konzentration des gekoppelten Antikörpers beträgt 1,27 und wird auf 1 mg/mL (~6,5 μM) mit PBS; 0,2 mM Na-Azid eingestellt und bei 4°C gelagert. Die Effizienz der Markierung berechnet sich wie folgt:

$$\text{Mol Farbstoff pro Mol Antikörper} = \frac{A_{679} \times \text{Verdünnungsfaktor}}{180\,00 \times \text{Proteinkonzentrartion M}}$$

wobei 184 000 $cm^{-1}M^{-1}$ der molare Extinktionskoeffizient des Alexa Fluor 680 Farbstoffes bei 679 nm ist. Die Ratio des Antikörper-Farbstoff-Konjugates beträgt 3,2.

Durchführung des Assays:

Die notwendigen Verdünnungen der verschiedenen Komponenten werden in 50 mM TSA-Puffer (50 mM Tris-HCl pH 7,75; 0,9 % NaCl; 0,05% $NaN_3$) durchgeführt. 40 wells einer UV-durchlässige Mikrotiterplatten (UVStar, Greiner) werden zunächst für 1 Std. bei RT mit einer 0,5 %igen BSA-Lösung inkubiert, um unspezifische Bindungen abzusättigen und anschließend mit einem Gemisch aus in Beispiel 17 dargestellten Nanoteilchen ($LaPO_4$: $Eu^{3+}$/IL-2 Konjugat), Alexa

Fluor 680-markiertem anti-hIL-2Rα-chain Antikörper und rekombinantem hIL-2sRα Protein (humaner IL-2 löslicher Rezeptor alpha, R&D Systems, Mineapolis, MN) mit einer jeweiligen Endkonzentration von 40 nM beladen. Zu 20 der wells wird in unterschiedlichen Konzentrationen unmarkiertes hIL-2 Protein gegeben, zu den anderen 20 ein für diesen Assay irrelevantes Protein. Die Konzentration wird jeweils um 50 nM erhöht, so dass eine Konzentrationsreihe von 0-950 nM getestet wird. Das Endvolumen der Reaktion beträgt jeweils 200 μl. Für 45 Min. wird im Dunklen bei RT auf einem Schüttler inkubiert. Die Signale werden mit einem Wallac 1420 Victor™ Multilabel Counter (Perkin-Elmer Life Sciences - Wallac Oy, Finnland) mit folgenden Einstellungen ausgelesen: Anregung: 340 nm, Emission: 665 nm, Zeitverzögerung (time delay): 50 μs, Zeitfenster (time window): 200 μs und Zyklus-Zeit (cycling time): 1000 μs. Für jeden Wert wird eine Doppelbestimmung durchgeführt und anhand der Ergebnisse mit dem irrelevanten Protein für unspezifische Bindungen korrigiert. Die gemessenen Werte werden gegen die eingesetzten Protein-Konzentration in einem Diagramm aufgetragen und ergeben eine Eichkurve, anhand derer Konzentrationen von humanem Interleukin-2, die in analoger Weise in humanen Körperproben gemessen werden, bestimmt werden können.

**<u>Beispiel 20</u>: Quantitative PCR-Bestimmung bakterieller DNA durch einen intramolekularen Energie Transfer mit den in Beispiel 15 dargestellten Nanoteilchen**

Die Primer und die Sonde für die quantitative DNA Bestimmung wurden spezifisch für das RNA Polymerase Gen von *Mycobacterium tuberculosis* ausgesucht und bei Interactiva (Ulm) hergestellt. Die Primer haben folgende Sequenzen: vorwärts: 5'-GGCCGGTGGTCGCCGCG-3', rückwärts: 5'-ACGTGACAGACCGCCGGGC -3'.

Assay zur quantitativen Bestimmung bakterieller DNA

Für die 50 μL PCR Reaktionen werden 50 nM der in Beispiel 15 dargestellten Nanopteilchen (Dabcyl-Oligonukleotid gekoppelt) als Sonde, jeweils 500 nM der beiden Primer, 2 U Amplitaq Gold DNA Polymerase (Perkin-Elmer), 250 μM dATP, 250 μM dCTP, 250 μM dGTP, 500 μM dUTP, 4 mM MgCl2, 50 mM KCl und 10 mM Tris-HCl pH 8,0 gemischt. Als DNA Template wird genomische *M. tuberculosis* DNA mit den gleichen Primern amplifiziert und mit Hilfe des Invitrogen Zero Blunt TOPO PCR Cloning Kit (Invitrogen BV/ NOVEX, Niederlande) in ein Plasmid kloniert. Um eine Standard-Kurve zu erstellen, werden 5 unterschiedliche Konzentrationen des DNA Plasmides von 1 pg bis 100 ng, eingesetzt sowie eine Reaktion ohne DNA Template. Für jede Konzentration werden 30 Reaktionen angesetzt, so dass, startend nach dem 15. Zyklus, nach jedem weiteren Zyklus eine Probe zur Messung im Spektrometer entnommen werden kann. Das Reaktionsvolumen beträgt 50 μL, die Amplifikation wird auf einem Thermocycler (PCR-System 2400, Perkin-Elmer) bei folgenden Reaktionsbedingungen durchgeführt: 10 Min. 95°C; 15 - 45 Zyklen mit 30 s bei 95°C, 45 s bei 56°C und 30 s bei 72°C. Die Proben werden in einem Fluoreszenz-Spektrometer (Fa. Jobin Yvon, Fluorolog 3) mit folgenden Einstellungen gemessen: Gepulste Anregung mit Anregungswellenlänge: 280 nm, Emission: 542 nm, Spaltbreite: 4 nm, Zeitverzögerung (time delay): 50 μs, Repetitionsrate ca. 25 Hz vermessen. Ebenso kann die Halbwertszeit der Terbium-Emissionslinie bestimmt werden. Dazu werden folgende Einstellungen verwendet: Anregung: 280 nm, Emission 542 nm, Spaltbreite 5nm; Integrationszeit: 0,1 ms. Während der Hybridisierung der Sonde an die Ziel-DNA findet kein intramolekularer FRET zwischen dem Nanoteilchen und dem Dabcyl statt. Bei ansteigender Konzentration der Ziel-DNA wird daher die Tb-Fluoreszenz des Nanoteilchens verglichen mit der Kontrolle ohne Template stärker, gleichzeitig verlängert sich die Halbwertszeit der Fluoreszenzlebensdauer des Nanoteilchens gegenüber der Kontrolle ohne Template DNA. Diese Differenzen beider Parameter können gegen die Anzahl der Zyklen zur Erstellung einer Eichkurve für jede DNA-Template Konzentration aufgetragen werden.

## Patentansprüche

1. Assay auf der Basis eines Resonanz-Energie-Transfers (RET), umfassend

- eine erste Molekülgruppe A, die mit mindestens einem Energie-Donor (Donor) markiert ist, und
- mindestens eine zweite Molekülgruppe B, die mit mindestens einem Energie-Akzeptor (Akzeptor) markiert ist,
- wobei der Donor ein Molekül oder Teilchen umfasst, das durch eine äußere Strahlungsquelle energetisch anregbar und fluoreszenzfähig ist,
- der Akzeptor ein Molekül oder Teilchen umfasst, das durch Energietransfer über den Donor unter teilweiser oder vollständiger Löschung der Donor-Fluoreszenz anregbar ist, und
- Donor und/oder Akzeptor <u>l</u>umineszierende <u>a</u>norganische <u>d</u>otierte Nanoteilchen (lad-Nanoteilchen) kristalliner Struktur mit einer Ausdehnung von ≤ 10 Nanometer umfassen, die nach einer energetischen Anregung elektromagnetische Strahlung mit einer Stokes-oder Antistokes-Verschiebung emittieren.

**2.** Assay gemäß Anspruch 1, **dadurch gekennzeichnet, dass** auch der Akzeptor fluoreszenzfähig ist.

**3.** Assay gemäß Anspruch 1, **dadurch gekennzeichnet, dass** der Akzeptor strahlunglos relaxiert.

**4.** Assay gemäß einem der vorstehenden Ansprüche, **dadurch gekennzeichnet, dass** es sich bei dem RET um einen Fluoreszenz-Resonanz-Energie-Transfer (FRET) handelt.

**5.** Assay gemäß einem der vorstehenden Ansprüche, **dadurch gekennzeichnet, dass** es sich bei dem RET um einen Förster-Transfer oder um einen Transfer unter Beteiligung von höheren Multipolordnungen handelt.

**6.** Assay gemäß einem der vorstehenden Ansprüche, **dadurch gekennzeichnet, dass** es sich bei dem RET um eine Migration von Ladungen oder Excitonen handelt.

**7.** Assay gemäß einem der vorstehenden Ansprüche, **dadurch gekennzeichnet, dass** der RET durch zeitaufgelöste oder kontinuierliche Messung der Änderung der Lumineszenzeigenschaften, insbesondere der Intensitätsänderung des Fluoreszenzlichts, spektralen Änderung des Fluoreszenzlichts oder Änderung der Zerfallszeit der lad-Nanoteilchen und/oder anderer Donoren und/oder Akzeptoren, insbesondere Chromophoren, qualitativ und/oder quantitativ erfasst werden kann.

**8.** Assay gemäß einem der vorstehenden Ansprüche, **dadurch gekennzeichnet, dass** der eine von Donor und Akzeptor lad-Nanoteilchen mit langer Fluoreszenz-Zerfallszeit, die vorzugsweise eine Halbwertszeit von mehr als 5 ns, insbesondere zwischen 1$\mu$s und 50 ms und besonders bevorzugt zwischen 100 $\mu$s und 10 ms aufweisen, umfasst und der jeweils andere entweder ebenfalls lad-Nanoteilchen, deren Fluoreszenz-Zerfallszeit kürzer als die der anderen lad-Nanoteilchen ist, oder einen molekularen organischen Chromophor umfasst.

**9.** Assay gemäß einem der vorstehenden Ansprüche, **dadurch gekennzeichnet, dass** das Wirtsmaterial der lad-Nanoteilchen Verbindungen des Typs XY enthält, wobei X ein Kation aus einem oder mehreren Elementen der Hauptgruppen 1a, 2a, 3a, 4a, der Nebengruppen 2b, 3b, 4b, 5b, 6b, 7b oder der Lanthaniden des Periodensystems ist und Y entweder ein mehratomiges Anion aus einem oder mehreren Element(en) der Hauptgruppen 3a, 4a, 5a, der Nebengruppen 3b, 4b, 5b, 6b, 7b und/oder 8b sowie Element(en) der Hauptgruppen 6a und/oder 7 oder ein einatomiges Anion aus der Hauptgruppe 5a, 6a oder 7a des Periodensystems ist.

**10.** Assay gemäß dem vorstehenden Anspruch, **dadurch gekennzeichnet, dass** das Wirtsmaterial der lad-Nanoteilchen Verbindungen aus der Gruppe der Sulfide, Selenide, Sulfoselenide, Oxysulfide, Borate, Aluminate, Gallate, Silikate, Germanate, Phosphate, Halophosphate, Oxide, Arsenate, Vanadate, Niobate, Tantalate, Sulfate, Wolframate, Molybdate, Alkalihalogenide sowie andere Halogenide oder Nitride enthält.

**11.** Assay gemäß einem der vorstehenden Ansprüche, **dadurch gekennzeichnet, dass** als Dotierung ein oder mehrere Elemente aus der Gruppe enthaltend Elemente der Hauptgruppen 1a, 2a oder Al, Cr, Tl, Mn, Ag, Cu, As, Nb, Nd, Ni, Ti, In, Sb, Ga, Si, Pb, Bi, Zn, Co und/oder Elemente der Lanthaniden verwendet werden.

**12.** Assay gemäß einem der vorstehenden Ansprüche, **dadurch gekennzeichnet, dass** auch Kombinationen von zwei oder mehreren dieser Elemente in unterschiedlichen relativen Konzentrationen zueinander als Dotierungsmaterial dienen.

**13.** Assay gemäß einem der vorstehenden Ansprüche, **dadurch gekennzeichnet, dass** die Konzentration des Dotierungsmaterials im Wirtsgitter zwischen $10^{-5}$ mol-% und 50 mol-%, bevorzugt zwischen 0,01 mol-% und 30 mol-%, besonders bevorzugt zwischen 0,1 mol-% und 20 mol-% beträgt.

**14.** Assay gemäß einem der vorstehenden Ansprüche, **dadurch gekennzeichnet, dass** LiI:Eu; NaI:Tl; CsI:Tl; CsI:Na; LiF:Mg; LiF:Mg,Ti; LiF:Mg,Na; $KMgF_3$:Mn; $Al_2O_3$:Eu; BaFCl:Eu; BaFCl:Sm; BaFBr:Eu; $BaFCl_{0,5}Br_{0,5}$:Sm; $BaY_2F_8$: A (A = Pr, Tm, Er, Ce); $BaSi_2O_5$:Pb; $BaMg_2Al_{16}O_{27}$:Eu; $BaMgAl_{14}O_{23}$:Eu; $BaMgAl_{10}O_{17}$:Eu; $BaMgAl_2O_3$:Eu; $Ba_2P_2O_7$:Ti; $(Ba,Zn,Mg)_3Si_2O_7$:Pb; $Ce(Mg,Ba)Al_{11}O_{19;}$ $Ce_{0,65}Tb_{0,35}MgAl_{11}O_{19:Ce,Tb;}$ $MgAl_{11}O_{19}$:Ce,Tb; $MgF_2$:Mn; MgS:Eu; MgS:Ce; MgS:Sm; MgS:(Sm,Ce); (Mg,Ca)S:Eu; $MgSiO_3$:Mn; 3,5MgO·0,5$MgF_2$·$GeO_2$:Mn; $MgWO_4$:Sm; $MgWO_4$:Pb; 6MgO·$As_2O_5$:Mn; $(Zn,Mg)F_2$:Mn; $(Zn_4Be)SO_4$:Mn; $Zn_2SiO_4$:Mn; $Zn_2SiO_4$:Mn,As; ZnO:Zn; ZnO:Zn,Si, Ga; $Zn_3(PO_4)_2$:Mn; ZnS:A (A = Ag, Al, Cu); (Zn,Cd)S:A (A = Cu, Al, Ag, Ni); $CdBO_4$:Mn; $CaF_2$:Mn; $CaF_2$:Dy; CaS: A A = Lanthanide, Bi); (Ca,Sr)S:Bi; $CaWO_4$:Pb; $CaWO_4$:Sm; $CaSO_4$:A (A = Mn, Lanthanide); $3Ca_3(PO_4)_2$·Ca(F, $Cl)_2$:Sb,$M_n$; $CaSiO_3$:Mn,Pb; $Ca_2Al_2Si_2O_7$:Ce; $(Ca,Mg)SiO_3$:Ce; $(Ca,Mg)SiO_3$:Ti; ZSrO·6$(B_2O_3)$·$SrF_2$:Eu; $3Sr_3$

$(PO_4)_2 \cdot CaCl_2$:Eu; $A_3(PO_4)_2 \cdot ACl_2$:Eu (A = Sr, Ca, Ba); $(Sr,Mg)_2P_2O_7$:Eu; $(Sr,Mg)_3(PO_4)_2$:Sn; SrS:Ce; SrS:Sm,Ce; SrS:Sm; SrS:Eu; SrS:Eu,Sm; SrS:Cu,Ag; $Sr_2P_2O_7$:Sn; $Sr_2P_2O_7$:Eu; $Sr_4Al_{14}O_{25}$:Eu; $SrGa_2S_4$:A (A = Lanthanide, Pb); $SrGa_2S_4$:Pb; $Sr_3Gd_2Si_6O_{18}$:Pb,Mn; $YF_3$:Yb,Er; $YF_3$:Ln (Ln = Lanthanide); $YLiF_4$:Ln (Ln = Lanthanide); $Y_3Al_5O_{12}$:Ln (Ln = Lanthanide); $YAl_3(BO_4)_3$:Nd,Yb; $(Y,Ga)BO_3$:Eu; $(Y,Gd)BO_3$:Eu; $Y_2Al_3Ga_2O_{12}$:Tb; $Y_2SiO_5$:Ln (Ln = Lanthanide); $Y_2O_3$:Ln (Ln = Lanthanide); $Y_2O_2S$:Ln (Ln = Lanthanide); $YVO_4$:A (A = Lanthanide, In); $Y(P,V)O_4$:Eu; $YTaO_4$:Nb; $YAlO_3$:A (A = Pr, Tm, Er, Ce); YOCl:Yb,Er; $LnPO_4$:Ce,Tb (Ln = Lanthanide oder Mischungen von Lanthaniden) ; $LuVO_4$:Eu; $GdVO_4$:Eu; $Gd_2O_2S$:Tb; $GdMgB_5O_{10}$:Ce,Tb; LaOBr:Tb; $La_2O_2S$:Tb; $LaF_3$:Nd,Ce; $BaYb_2F_8$:Eu; $NaYF_4$:Yb,Er; $NaGdF_4$:Yb,Er; $NaLaF_4$:Yb,Er; $LaF_3$:Yb,Er,Tm; $BaYF_5$:Yb,Er; $Ga_2O_3$:Dy; GaN:A (A = Pr, Eu, Er, Tm); $Bi_4Ge_3O_{12}$; $LiNbO_3$:Nd,Yb; $LiNbO_3$:Er; $LiCaAlF_6$:Ce; $LiSrAlF_6$:Ce; $LiLuF_4$:A (A = Pr, Tm, Er, Ce); $Li_2B_4O_7$:Mn, $SiO_x$:Er,Al ($0 \leq x \leq 2$) als Material für die lad-Nanoteilchen verwendet wird.

**15.** Assay gemäß einem der vorstehenden Ansprüche, **dadurch gekennzeichnet, dass** $YVO_4$:Eu, $YVO_4$:Sm, $YVO_4$:Dy, $LaPO_4$:Eu, $LaPO_4$:Ce, $LaPO_4$:Ce,Tb, $LaPO_4$:Ce,Dy, $LaPO_4$:Ce,Nd, ZnS:Tb, ZnS:$TbF_3$, ZnS:Eu, ZnS:$EuF_3$, $Y_2O_3$:Eu, $Y_2O_2S$:Eu, $Y_2SiO_5$:Eu, $SiO_2$:Dy, $SiO_2$:Al, $Y_2O_3$:Tb, CdS:Mn, ZnS:Tb, ZnS:Ag oder ZnS:Cu als Material für die lad-Nanoteilchen verwendet wird.

**16.** Assay gemäß einem der vorstehenden Ansprüche, **dadurch gekennzeichnet, dass** Material mit einer kubischen Gitterstruktur des Wirtsgitters für die lad-Nanoteilchen verwendet wird.

**17.** Assay gemäß einem der vorstehenden Ansprüche, **dadurch gekennzeichnet, dass** $MgF_2$:Mn; ZnS:Mn, ZnS:Ag, ZnS:Cu, $CaSiO_3$:Ln, CaS:Ln, CaO:Ln, ZnS:Ln, $Y_2O_3$:Ln, oder $MgF_2$:Ln (Ln = Lanthaniden) als Material für die lad-Nanoteilchen verwendet wird.

**18.** Assay gemäß einem der vorstehenden Ansprüche, **dadurch gekennzeichnet, dass** Donor und/oder Akzeptor lad-Nanoteilchen umfassen, die die elektromagnetische Strahlung mit einer Stokes- oder Antistokes-Verschiebung nach einer energetischen Anregung mit elektromagnetischer Strahlung mit Wellenlängen im Bereich des infraroten Lichts, des sichtbaren Lichts, des UV, des Röntgenlichts oder der $\gamma$-Strahlung oder Teilchenstrahlung wie Elektronenstrahlung emittieren.

**19.** Assay gemäß einem der vorstehenden Ansprüche, **dadurch gekennzeichnet, dass** der Donor lad-Nanoteilchen umfasst und der Akzeptor aus einem leitfähigen Material, vorzugsweise aus einem Metall, insbesondere Gold, Silber oder Platin, einem leitfähigen Oxid, insbesondere Indium-Zinn-Oxid (ITO) oder einem leitfähigen Polymer, besteht, das bevorzugt in partikulärer Form als Nano- oder Mikroteilchen oder als ebene, optional strukturierte, Oberfläche vorliegt.

**20.** Assay gemäß einem der vorstehenden Ansprüche, **dadurch gekennzeichnet, dass** es sich um einen homogenen Assay ohne Wasch- oder Separierungsschritte handelt.

**21.** Assay gemäß einem der vorstehenden Ansprüche, **dadurch gekennzeichnet, dass** der Assay ein homogener Immunoassay zum Nachweis mindestens eines Analyten, insbesondere mindestens eines monoklonalen oder polyklonalen Antikörpers, Proteins, Peptids, Oligonukleotids, Nukleinsäure, Oligo- oder Polysaccharids, Haptens oder niedermolekularen synthetischen oder natürlichen Antigens, in einer Probe, insbesondere in Abstrichen, Sputum, Organpunktat, Biopsien, Sekreten, Liquor, Galle, Blut, Lymphflüssigkeit, Urin und/oder Stuhl, ist.

**22.** Assay gemäß einem der vorstehenden Ansprüche, **dadurch gekennzeichnet, dass** die Oberfläche des oder der lad-Nanoteilchen so präpariert ist, dass Affinitätsmoleküle daran ankoppeln können.

**23.** Assay nach Anspruch 22, **dadurch gekennzeichnet, dass** die Oberfläche der lad-Nanoteilchen chemisch modifiziert ist und/oder reaktive Gruppen und/oder kovalent oder nicht-kovalent gebundene Verbindungsmoleküle aufweist, wobei die angebundenen Verbindungsmoleküle ihrerseits wieder reaktive Gruppen aufweisen können.

**24.** Assay nach Anspruch 23, **dadurch gekennzeichnet, dass** die reaktiven Gruppen Aminogruppen, Carbonsäuregruppen, Thiole, Thioether, Disulfide, Imidazole, Guanidine, Hydroxylgruppen, Indole, vicinale Diole, Aldehyde, alpha-Haloacetylgruppen, N-Maleimide, Quecksilberorganyle, Arylhalogenide, Säureanhydride, Isocyanate, Isothiocyanate, Sulfonsäurehalogenide, Imidoester, Diazoacetate, Diazoniumsalze, 1,2-Diketone, alpha-beta-ungesättigte Carbonylverbindungen, Phosphonsäuren, Phosphorsäureester, Sulfonsäuren, Azolide oder Derivate der genannten Gruppen sind.

**25.** Assay nach Anspruch 23 oder 24, **dadurch gekennzeichnet, dass** die Verbindungsmoleküle Nukleinsäuremoleküle, Phosphonsäurederivate, Ethylenglykol, primäre Alkohole, Aminderivate, Polymere oder Copolymere, polymerisierbare Kopplungsagenzien, Silica-Hüllen und kettenförmige Moleküle mit einer der Oberfläche der lad-Nanoteilchen entgegengesetzten Polarität sind.

**26.** Assay nach Anspruch 25, **dadurch gekennzeichnet dass** polymerisierbare Kopplungsagenzien Diacetylene, Styrolbutadiene, Vinylacetat, Acrylate, Acrylamide, Vinyle, Styrole, Silikonoxide, Boroxide, Phosphoroxide, Borate, Pyrrole, Polypyrrole und Phosphate sowie Polymere von zumindest einigen der besagten Agenzien sind.

**27.** Assay nach Anspruch 22 bis 26, **dadurch gekennzeichnet, dass** die Affinitätsmoleküle Proteine, Peptide, Oligonukleotide oder andere Nukleinsäuremoleküle oder nukleinsäureähnliche Moleküle, wie PNAs oder Morpholinos, Oligo- oder Polysaccharide, Haptene, wie Biotin oder Digoxin oder niedermolekulare synthetische oder natürliche Antigene oder Epitope sind.

**28.** Assay nach Anspruch 22 bis 27, **dadurch gekennzeichnet, dass** die Affinitätsmoleküle in Wechselwirkung mit Zielmolekülen treten können.

**29.** Assay gemäß Anspruch 28, **dadurch gekennzeichnet, dass** das Zielmolekül ein Enzym, ein Antikörper, ein Nukleinsäure bindendes Molekül, eine Nukleinsäure, ein Polynukleotid oder Morpholino ist.

**30.** Assay gemäß Anspruch 29, **dadurch gekennzeichnet, dass** das Enzym Endonuklease, Protease, Kinase, Phosphatase oder ein Aminosäure oder Nukleinsäure modifizierendes oder spaltendes Enzym ist.

**31.** Assay gemäß einem der Ansprüche 1 bis 30, **dadurch gekennzeichnet, dass** die Molekülgruppen A und B Bestandteil ein und desselben Moleküls sind.

**32.** Assay gemäß einem der vorhergehenden Ansprüche, **dadurch gekennzeichnet, dass** die Molekülgruppen A und B an dasselbe Affinitätsmolekül ankoppeln können.

**33.** Assay gemäß Anspruch 31, **dadurch gekennzeichnet, dass** eine Interaktion des Affinitätsmoleküls mit dem Zielmolekül eine Änderung des räumlichen Abstandes der Molekülgruppen A und B zur Folge hat.

**34.** Assay gemäß einem der vorhergehenden Ansprüche, **dadurch gekennzeichnet, dass** er zur Quantifizierung von Nukleinsäuren verwendet wird.

**35.** Assay gemäß einem der Ansprüche 1 bis 30, **dadurch gekennzeichnet, dass** die Molekülgruppen A und B Bestandteile unterschiedlicher Moleküle sind.

**36.** Assay gemäß Anspruch 35, **dadurch gekennzeichnet, dass** die Molekülgruppen A und B jeweils an eigene Affinitätsmoleküle angekoppelt sind.

**37.** Assay gemäß Anspruch 36, **dadurch gekennzeichnet, dass** die den Molekülgruppen A und B zugeordneten Affinitätsmoleküle mit demselben Zielmolekül spezifisch interaktionsfähig sind.

**38.** Assay gemäß Anspruch 37, **dadurch gekennzeichnet, dass** die den Molekülgruppen A und B zugeordneten Affinitätsmoleküle spezifisch miteinander interaktionsfähig sind.

**39.** Assay gemäß Anspruch 37 oder 38, **dadurch gekennzeichnet, dass** eine Interaktion der den Molekülgruppen A und B zugeordneten Affinitätsmoleküle mit dem gemeinsamen Zielmolekül oder miteinander eine Änderung des räumlichen Abstandes der Molekülgruppen A und B zur Folge hat.

**40.** Verfahren zum Nachweis eines Zielmoleküls, enthaltend die Schritte

- Bereitstellung eines Assays nach einem der Ansprüche 1 bis 34,
wobei in dem Assay die Molekülgruppen A und B Bestandteil ein und desselben Moleküls sind und an dasselbe Affinitätsmolekül ankoppeln, das mit einem spezifischen Zielmolekül interaktionsfähig ist, und eine solche Interaktion eine Änderung des Abstandes zwischen den Molekülgruppen A und B bewirkt,
- Zugabe einer Probe enthaltend das Zielmolekül zum Assay,

- Anregung des Assays mit Probe durch eine Quelle für elektromagnetische oder partikuläre Strahlung,
- Messung der durch das Assay mit Probe emittierten elektromagnetischen Strahlung,
wobei die Intensität oder das Spektrum der emittierten elektromagnetischen Strahlung oder der zeitliche Verlauf der Emission der elektromagnetischen Strahlung ein Maß für die Menge von Zielmolekül in der Probe ist.

**41.** Verfahren zum Nachweis eines Zielmoleküls, enthaltend die Schritte

- Bereitstellung eines Assays nach einem der Ansprüche 1 bis 30 oder 35 bis 39,
- wobei die Molekülgruppen A und B Bestandteile unterschiedlicher Moleküle sind und

die den Molekülgruppen A und B zugeordneten Affinitätsmoleküle mit ein und demselben Zielmolekül spezifisch interaktionsfähig sind oder
die den Molekülgruppen A und B zugeordneten Affinitätsmoleküle spezifisch miteinander interaktionsfähig sind, und in beiden Fällen eine Interaktion eine Änderung des Abstandes zwischen den Molekülgruppen A und B bewirkt,

- Zugabe einer Probe enthaltend das Zielmolekül zum Assay,
- Anregung des Assays mit Probe durch eine Quelle für elektromagnetische oder partikuläre Strahlung,
- Messung der durch das Assay mit Probe emittierten elektromagnetischen Strahlung,
wobei die Intensität oder das Spektrumder emittierten elektromagnetischen Strahlung oder der zeitliche Verlauf der Emission der elektromagnetischen Strahlung ein Maß für die Menge von Zielmolekül in der Probe ist.

## Claims

**1.** An assay based on a resonance energy transfer (RET) which comprises
a first molecule group A which is labeled with at least one energy donor (donor), and
at least a second molecule group B which is labeled with at least one energy acceptor (acceptor),
where the donor comprises a molecule or particle which can be energetically excited by an external radiation source and is capable of fluorescence,
the acceptor comprises a molecule or particle which can be excited by energy transfer by way of the donor, with partial or complete quenching of the donor fluorescence, and
donor and/or acceptor comprise luminescent inorganic doped nanoparticles (lid nanoparticles) of crystalline structure which have a breadth of ≤ 10 nanometers and which, after an energetic excitation, emit electromagnetic radiation with a Stokes shift or an anti-Stokes shift.

**2.** Assay according to Claim 1, **characterized in that** the acceptor is also capable of fluorescence.

**3.** Assay according to Claim 1, **characterized in that** the acceptor relaxes in a radiationless manner.

**4.** Assay according to one of the preceding claims, **characterized in that** the RET is a fluorescence resonance energy transfer (FRET).

**5.** Assay according to one of the preceding claims, **characterized in that** the RET is a Förster transfer or a transfer involving higher multipole orders.

**6.** Assay according to one of the preceding claims, **characterized in that** the RET is a migration of charges or excitons.

**7.** Assay according to one of the preceding claims, **characterized in that** the RET can be qualitatively and/or quantitatively recorded by the time-resolved or continuous measurement of the change in the luminescence properties, in particular the change in the intensity of the fluorescent light, the change in the spectrum of the fluorescent light or the change in the decay time of the lid nanoparticles and/or of other donors and/or acceptors, in particular chromophores.

**8.** Assay according to one of the preceding claims, **characterized in that** either the donor or acceptor comprises lid nanoparticles which have a long fluorescence decay time and which preferably exhibit a half life of more than 5 ns, particularly between 1 μs and 50 ms, and particularly preferably between 100 μs and 10 ms, and the other one of the two either also comprises lid nanoparticles whose fluorescence decay time is shorter than that of the other lid nanoparticles or comprises a molecular organic chromophore.

**9.** Assay according to one of the preceding claims, **characterized in that** the host material of the lid nanoparticles contains compounds of the type XY, where X is a cation consisting of one or more elements of the main groups 1a, 2a, 3a or 4a, of the subgroups 2b, 3b, 4b, 5b, 6b or 7b, or of the lanthanides, of the periodic system, and Y is either a polyatomic anion consisting of one or more element (s) of the main groups 3a, 4a or 5a, or of the subgroups 3b, 4b, 5b, 6b 7b and/or 8b, and also element(s) of the main groups 6a and/or 7, or else is a monoatomic anion from the main group 5a, 6a or 7a of the periodic system.

**10.** Assay according to the preceding claim, **characterized in that** the host material of the lid nanoparticles contains compounds from the group of the sulfides, selenides, sulfoselenides, oxysulfides, borates, aluminates, gallates, silicates, germanates, phosphates, halophosphates, oxides, arsenates, vanadates, niobates, tantalates, sulfates, tungstates, molybdates, alkali metal halides and other halides, or nitrides.

**11.** Assay according to one of the preceding claims, **characterized in that** one or more elements from the group containing elements of the main groups 1a or 2a, or Al, Cr, Tl, Mn, Ag, Cu, As, Nb, Nd, Ni, Ti, In, Sb, Ga, Si, Pb, Bi, Zn or Co, and/or elements of the lanthanides, is/are used as doping.

**12.** Assay according to one of the preceding claims, **characterized in that** combinations of two or more of these elements, in different relative concentrations to each other, are also used as doping material.

**13.** Assay according to one of the preceding claims, **characterized in that** the concentration of the doping material in the host lattice is between $10^{-5}$ mol% and 50 mol%, preferably between 0.01 mol% and 30 mol%, particularly preferably between 0.1 mol% and 20 mol%.

**14.** Assay according to any one of the preceding claims, **characterized in that** LiI:Eu; NaI:Tl; CsI:Tl; CsI:Na; Lif:Mg; LiF:Mg,Ti; LiF:Mg,Na; $KMgF_3$:Mn; $Al_2O_3$:Eu; BaFCl:Eu; BaFCl:Sm; BaFBr:Eu; $BaFCl_{0.5}Br_{0.5}$:Sm; $BaY_2F_8$:A (A = Pr, Tm, Er, Ce); $BaSi_2O_5$:Pb; $BaMg_2Al_{16}O_{27}$:Eu; $BaMgAl_{14}O_{23}$:Eu; $BaMgAl_{10}O_{17}$:Eu; $BaMgAl_2O_3$:Eu; $Ba_2P_2O_7$:Ti; $(Ba,Zn,Mg)_3Si_2O_7$:Pb; $Ce(Mg,Ba)Al_{11}O_{19}$; $Ce_{0.65}Tb_{0.35}MgAl_{11}O_{19:Ce,Tb}$; $MgAl_{11}O_{19}$:Ce,Tb; $MgF_2$:Mn; MgS:Eu; MgS:Ce; MgS:Sm; MgS:(Sm,Ce); (Mg,Ca)S:Eu; $MgSiO_3$:Mn; $3.5MgO \cdot 0.5MgF_2 \cdot GeO_2$:Mn; $MgWO_4$:Sm; $MgWO_4$: Pb, $6MgO \cdot As_2O_5$:Mn; $(Zn,Mg)F_2$:Mn; $(Zn_4Be)SO_4$:Mn; $Zn_2SiO_4$:Mn; $Zn_2SiO_4$:Mn,As; ZnO:Zn; ZnO:Zn, Si,Ga; $Zn_3(PO_4)_2$:Mn; ZnS:A (A = Ag, Al, Cu); (Zn,Cd)S:A (A = Cu, Al, Ag, Ni); $CdBO_4$:Mn; $CaF_2$:Mn; $CaF_2$:Dy; CaS:A (A = lanthanides, Bi); (Ca,Sr)S:Bi; $CaWO_4$:Pb; $CaWO_4$:Sm; $CaSO_4$:A (A = Mn, lanthanides); $3Ca_3(PO_4)_2 \cdot Ca(F,Cl)_2$:Sb, $M_n$; $CaSiO_3$:Mn,Pb; $Ca_2Al_2Si_2O_7$:Ce; $(Ca,Mg)SiO_3$:Ce; $(Ca,Mg)SiO_3$:Ti; $2SrO \cdot 6(B_2O_3) \cdot SrF_2$:Eu; $3Sr_3(PO_4)_2 \cdot CaCl_2$: Eu; $A_3(PO_4)_2 \cdot ACl_2$:Eu (A = Sr, Ca, Ba); $(Sr,Mg)_2P_2O_7$:Eu; $(Sr,Mg)_3(PO_4)_2$:Sn; SrS:Ce; SrS:Sm,Ce; SrS:Sm; SrS: Eu; SrS:Eu,Sm; SrS:Cu,Ag; $Sr_2P_2O_7$:Sn; $Sr_2P_2O_7$:Eu; $Sr_4Al_{14}O_{25}$:Eu; $SrGa_2S_4A$ (A = lanthanides, Pb); $SrGa_2S_4$: Pb; $Sr_3Gd_2Si_6O_{18}$:Pb,Mn; $YF_3$:Yb,Er; $YF_3$:Ln (Ln = lanthanides); $YLiF_4$:Ln (Ln = lanthanides); $Y_3Al_5O_{12}$:Ln (Ln = lanthanides); $YAl_3(BO_4)_3$:Nd,Yb; $(Y,Ga)BO_3$:Eu; $(Y,Gd)BO_3$:Eu; $Y_2Al_3Ga_2O_{12}$:Tb; $Y_2SiO_5$:Ln (Ln = lanthanides); $Y_2O_3$:Ln (Ln = lanthanides); $Y_2O_2S$:Ln (Ln = lanthanides); $YVO_4$:A (A = lanthanides, In); $Y(P,V)O_4$:Eu; $YTaO_4$:Nb; $YAlO_3$:A (A = Pr, Tm, Er, Ce); YOCl:Yb, Er; $LnPO_4$: (LnCe,Tb = lanthanides or mixtures of lanthanides); $LuVO_4$:Eu; $GdVO_4$:Eu; $Gd_2O_2S$:Tb; $GdMgB_5O_{10}$:Ce,Tb; LaOBr:Tb; $La_2O_2S$:Tb; $LaF_3$Nd, Ce; $BaYb_2F_8$:Eu; $NaYF_4$:Yb,Er; $NaGdF_4$:Yb,Er; $NaLaF_4$:Yb,Er; $LaF_3$:Yb,Er,Tm; $BaYF_5$:Yb,Er; $Ga_2O_3$:Dy; GaN:A (A = Pr, Eu, Er, Tm); $Bi_4Ge_3O_{12}$; $LiNbO_3$:Nd,Yb; $LiNbO_3$:Er; $LiCaAlF_6$:Ce; $LiSrAlF_6$:Ce; $LiLuF_4$:A (A = Pr, Tm, Er, Ce); $Li_2B_4O_7$:Mn, $SiO_x$:Er,Al ($O \leq x \leq 2$) is used as material for the lid nanoparticles.

**15.** Assay according to one of the preceding claims, **characterized in that** $YVO_4$:Eu, $YVO_4$:Sm, $YVO_4$:Dy, $LaPO_4$:Eu, $LaPO_4$:Ce, $LaPO_4$:Ce,Tb, $LaPO_4$:Ce,Dy, $LaPO_4$:Ce,Nd, ZnS:Tb, ZnS:$TbF_3$, ZnS:Eu, ZnS:$EuF_3$, $Y_2O_3$:Eu, $Y_2O_2S$: Eu, $Y_2SiO_5$:Eu, $SiO_2$:Dy, $SiO_2$:Al, $Y_2O_3$:Tb, CdS:Mn, ZnS:Tb, ZnS:Ag or ZnS:Cu is used as the material for the lid nanoparticles.

**16.** Assay according to one of the preceding claims, **characterized in that** material in which the host lattice has a cubic structure is used for the lid nanoparticles.

**17.** Assay according to one of the preceding claims, **characterized in that** $MgF_2$:Mn, ZnS:Mn, ZnS:Ag, ZnS:Cu, CaSiO3: Ln, CaS:Ln, CaO:Ln, ZnS:Ln, $Y_2O_3$:Ln, or $MgF_2$:Ln (Ln = lanthanides) is used as material for the lid nanoparticles.

**18.** Assay according to one of the preceding claims, **characterized in that** donor and/or acceptor comprise lid nanoparticles which, after energetic excitation with electromagnetic radiation with wavelengths in the range of infrared light, of visible light, of UV, of X-ray light or of $\gamma$-radiation, or particle radiation, such as electron radiation, emit electromagnetic radiation with a Stokes shift or anti-Stokes shift.

**19.** Assay according to one of the preceding claims, **characterized in that** the donor comprises lid nanoparticles and the acceptor consists of a conducting material, preferably of a metal, in particular gold, silver or platinum, a conducting oxide, in particular indium tin oxide (ITO) or a conducting polymer which is preferably present in particulate form as nanoparticles or microparticles or as a planar, optionally structured, surface.

**20.** Assay according to one of the preceding claims, **characterized in that** it is a homogeneous assay without any washing or separating steps.

**21.** Assay according to one of the preceding claims, **characterized in that** the assay is a homogeneous immunoassay detecting at least one analyte, in particular at least one monoclonal or polyclonal antibody, protein, peptide, oligonucleotide, nucleic acid, oligosaccharide, polysaccharide, hapten or low molecular weight synthetic or natural antigen in a sample, in particular in smears, sputum, organ punctate, biopsies, secretions, spinal fluid, bile, blood, lymph fluid, urine and/or feces.

**22.** Assay according to one of the preceding claims, **characterized in that** the surface of the lid nanoparticle(s) is prepared such that affinity molecules can be coupled to it.

**23.** Assay according to Claim 22, **characterized in that** the surface of the lid nanoparticles is chemically modified and/or exhibits reactive groups and/or covalently or noncovalently bound connecting molecules, with the bound connecting molecules being able, for their part, once again to exhibit reactive groups.

**24.** Assay according to Claim 23, **characterized in that** the reactive groups are amino groups, carboxylic acid groups, thiols, thioethers, disulfides, imidazoles, guanidines, hydroxyl groups, indoles, vicinal diols, aldehydes, alpha-haloacetyl groups, N-maleimides, mercurides, aryl halides, acid anhydrides, isocyanates, isothiocyanates, sulfonyl halides, imidoesters, diazoacetates, diazonium salts, 1,2-diketones, alpha-beta-unsaturated carbonyl compounds, phosphonic acids, phosphoric acid esters, sulfonic acids or azolides, or derivatives of said groups.

**25.** Assay according to Claim 23 or 24, **characterized in that** the connecting molecules are nucleic acid molecules, phosphonic acid derivatives, ethylene glycol, primary alcohols, amine derivatives, polymers or copolymers, polymerizable coupling agents, silica shells and catenate molecules having a polarity which is opposite to that of the surface of the lid nanoparticles.

**26.** Assay according to Claim 25, **characterized in that** polymerizable coupling agents are diacetylenes, styrenebutadienes, vinyl acetate, acrylates, acrylamides, vinyls, styrenes, silicone oxides, boron oxides, phosphorus oxides, borates, pyrroles, polypyrroles and phosphates, and also polymers of at least some of said agents.

**27.** Assay according to Claim 22 to 26, **characterized in that** the affinity molecules are proteins, peptides, oligonucleotides or other nucleic acid molecules or nucleic acid-like molecules, such as PNAs or morpholinos, oligosaccharides or polysaccharides, haptens, such as biotin or digoxin, or low molecular weight synthetic or natural antigens or epitopes.

**28.** Assay according to Claim 22 to 27, **characterized in that** the affinity molecules are able to enter into interaction with target molecules.

**29.** Assay according to Claim 28, **characterized in that** the target molecule is an enzyme, an antibody, a nucleic acid-binding molecule, a nucleic acid, a polynucleotide or a morpholino.

**30.** Assay according to Claim 29, **characterized in that** the enzyme is endonuclease, protease, kinase or phosphatase or an amino acid- or nucleic acid-modifying or cleaving enzyme.

**31.** Assay according to one of Claims 1 to 30, **characterized in that** the molecule groups A and B are constituents of one and the same molecule.

**32.** Assay according to one of the preceding claims, **characterized in that** the molecule groups A and B are able to couple to the same affinity molecule.

**33.** Assay according to Claim 31, **characterized in that** an interaction of the affinity molecule with the target molecule results in a change in the spatial separation of molecule groups A and B.

**34.** Assay according to one of the preceding claims, **characterized in that** it is used for quantifying nucleic acids.

**35.** Assay according to one of Claims 1 to 30, **characterized in that** the molecule groups A and B are constituents of different molecules.

**36.** Assay according to Claim 35, **characterized in that** the molecule groups A and B are in each case coupled to their own affinity molecules.

**37.** Assay according to Claim 36, **characterized in that** the affinity molecules which are assigned to molecule groups A and B are able to interact specifically with the same target molecule.

**38.** Assay according to Claim 37, **characterized in that** the affinity molecules which are assigned to molecule groups A and B are able to interact specifically with each other.

**39.** Assay according to Claim 37 or 38, **characterized in that** an interaction of the affinity molecules which are assigned to molecule groups A and B with the common target molecule or with each other result in a change in the spatial separation of molecule groups A and B.

**40.** Method for detecting a target molecule, comprising the steps of

- providing an assay according to one of Claims 1 to 34,
where, in the assay, the molecule groups A and B are constituents of one and the same molecule and couple to the same affinity molecule, which is capable of interacting with a specific target molecule, and such an interaction brings about a change in the separation between the molecule groups A and B,
- adding a sample containing the target molecule to the assay,
- exciting the assay containing the sample with a source of electromagnetic or particulate radiation,
- measuring the electromagnetic radiation emitted by the assay containing the sample,
where the intensity or the spectrum of the emitted electromagnetic radiation, or the chronological course of the emission of the electromagnetic radiation, is a measure of the quantity of target molecule in the sample.

**41.** Method for detecting a target molecule, comprising the steps of

- providing an assay according to one of Claims 1 to 30 or 35 to 39,
- where the molecule groups A and B are constituents of different molecules, and
the affinity molecules which are assigned to molecule groups A and B are capable of specifically interacting with one and the same target molecule, or
the affinity molecules which are assigned to molecule groups A and B are capable of specifically interacting with each other,
and in both cases an interaction brings about a change in the separation between molecule groups A and B,
- adding a sample containing the target molecule to the assay,
- exciting the assay containing the sample with a source of electromagnetic or particulate radiation,
- measuring the electromagnetic radiation emitted by the assay containing the sample,
where the intensity or the spectrum of the emitted electromagnetic radiation, or the chronological course of the emission of the electromagnetic radiation, is a measure of the quantity of target molecule in the sample.

**Revendications**

**1.** Analyse à base d'un transfert d'énergie par résonance (RET), comprenant :

- un premier groupe moléculaire A, qui est marqué avec au moins un donneur d'énergie (donneur), et
- au moins un deuxième groupe moléculaire B, qui est marqué avec au moins un accepteur d'énergie (accepteur),
- où le donneur comprend une molécule ou des particules, qui peuvent être excitées énergétiquement par une source extérieure de rayonnement et peut émettre une fluorescence,
- l'accepteur comprend une molécule ou des particules, qui peuvent être excitées par transfert d'énergie depuis le donneur, avec extinction partielle ou complète de la fluorescence du donneur,
- le donneur et/ou l'accepteur comprennent des nanoparticules dopées inorganiques luminescentes (nanoparticules dil) de structure cristalline avec une dimension ≤ 10 nm, qui après une excitation énergétique, émettent

un rayonnement électromagnétique avec un déplacement Stokes ou anti-Stokes.

2. Analyse suivant la revendication 1, **caractérisée en ce que** l'accepteur est capable de fluorescence.

3. Analyse suivant la revendication 1, **caractérisée en ce que** l'accepteur relaxe sans rayonnement.

4. Analyse suivant l'une des revendications précédentes, **caractérisée en ce que** le RET consiste en un transfert d'énergie par résonance en fluorescence (FRET).

5. Analyse suivant l'une des revendications précédentes, **caractérisée en ce que** le RET consiste en un transfert de Föster ou en un transfert avec participation d'ordres supérieurs de multipoles.

6. Analyse suivant l'une des revendications précédentes, **caractérisée en ce que** le RET consiste en une migration de charges ou d'excitons.

7. Analyse suivant l'une des revendications précédentes, **caractérisée en ce que** le RET peut être saisi de manière qualitative ou quantitative, par mesure temporaire ou continue de la modification des propriétés de luminescence, en particulier de la modification d'intensité de la lumière fluorescente, la modification spectrale de la lumière fluorescente ou la modification du temps de décroissance des nanoparticules dil et/ou d'autres donneurs et/ou accepteurs, en particulier des chromophores.

8. Analyse suivant l'une des revendications précédentes, **caractérisée en ce que** l'un des donneurs ou accepteurs comprend des nanoparticules dil avec temps de décroissance de fluorescence long, qui présente de préférence un temps de demi-vie de plus de 5 ns, en particulier allant de 1 $\mu$s à 50 ms et de manière particulièrement préférée, de 100 $\mu$s à 10 ms et chaque autre comprend également des nanoparticules dil, dont le temps de décroissance de fluorescence est plus court que celui des autres nanoparticules dil, ou un chromophore organique moléculaire.

9. Analyse suivant l'une des revendications précédentes, **caractérisée en ce que** le matériau-hôte des nanoparticules dil contient des composés de type XY, où X est un cation d'un ou de plusieurs éléments des groupes principaux 1a, 2a, 3a, 4a, des groupes secondaires 2b, 3b, 4b, 5b, 6b, 7b ou des lanthanides du système périodique et Y est un anion polyatomique d'un ou de plusieurs éléments des groupes principaux 3a, 4a, 5a, des groupes secondaires 3b, 4b, 5b, 6b, 7b et/ou 8b ainsi que des éléments des groupes principaux 6a et/ou 7 ou un anion monoatomique du groupe principal 5a, 6a ou 7a du système périodique.

10. Analyse suivant l'une des revendications précédentes, **caractérisée en ce que** le matériau-hôte des nanoparticules dil contient des composés du groupe des sulfures, sélénures, sulfosélénures, oxysulfures, borates, aluminates, gallates, silicates, germanates, phosphates, halophosphates, oxydes, arséniates, vanadates, niobates, tantalates, sulfates, tungstates, molybdates, halogénures alcalins ainsi que d'autres halogénures ou nitrures.

11. Analyse suivant l'une des revendications précédentes, **caractérisée en ce que** comme dopage, on utilise un ou plusieurs éléments du groupe contenant les éléments des groupes principaux 1a, 2a ou Al, Cr, Tl, Mn, Ag, Cu, As, Nb, Nd, Ni, Ti, In, Sb, Ga, Si, Pb, Bi, Zn, Co et/ou des éléments des lanthanides.

12. Analyse suivant l'une des revendications précédentes, **caractérisée en ce que** des combinaisons de deux ou plusieurs de ces éléments en différentes concentrations relatives l'une par rapport à l'autre servent comme matériau de dopage.

13. Analyse suivant l'une des revendications précédentes, **caractérisée en ce que** la concentration de matériau de dopage dans le réseau hôte se situe dans l'intervalle allant de $10^{-5}$% en moles à 50% en moles, de préférence de 0,01% en moles à 30% en moles, de manière particulièrement préférée, de 0,1% en moles à 20% en moles.

14. Analyse suivant l'une des revendications précédentes, **caractérisée en ce que** l'on utilise comme matériau pour les nanoparticules dil, LiI:Eu ; NaI:Tl ; CsI:Tl ; CsI:Na ; LiF:Mg ; LiF:Mg,Ti ; LiF:Mg,Na ; $KMgF_3$:Mn, $Al_2O_3$:Eu ; BaFCl Eu ; BaFCl:Sm ; BaFBr : Eu ; $BaFCl_{0,5}Br_{0,5}$:Sm ; $BaY_2F_8$:A (A = Pr, Tm, Er, Ce) ; $BaSi_2O_5$:Pb ; $BaMg_2Al_{16}O_{27}$:Eu ; $BaMgAl_{14}O_{23}$:Eu ; $BaMgAl_{10}O_{17}$:Eu ; $BaMgAl_2O_3$:Eu ; $Ba_2P_2O_7$:Ti ; $(Ba,Zn,Mg)_3Si_2O_7$:Pb ; Ce $(Mg,Ba)Al_{11}O_{19}$ ; $Ce_{0,65}Tb_{0,35}MgAl_{11}O_{19}$:Ce,Tb ; $MgAl_{11}O_{19}$:Ce,Tb ; $MgF_2$:Mn ; MgS:Eu ; MgS:Ce ; MgS:Sm ; MgS:(Sm,Ce) ; (Mg, Ca)S:Eu ; $MgSiO_3$:Mn ; 3,5MgO.0,5$MgF_2$.$GeO_2$:Mn ; $MgWO_4$:Sm ; $MgWO_4$:Pb ; 6MgO.$As_2O_5$:Mn ; $(Zn,Mg)F_2$:Mn ; $(Zn_4Be)SO_4$:Mn ; $Zn_2SiO_4$:Mn ; $Zn_2SiO_4$:Mn,As ; ZnO:Zn ; ZnO:Zn,Si,Ga ; $Zn_3(PO_4)_2$:Mn ; ZnS:A (A = Ag, Al, Cu) ;

(Zn,Cd)S:A (A = Cu, Al, Ag, Ni) ; $CdBO_4$:Mn ; $CaF_2$:Mn ; $CaF_2$:Dy ; CaS:A (A = lanthanide, Bi) ; (Ca,Sr)S:Bi ; $CaWO_4$:Pb ; $CaWO_4$:Sm ; $CaSO_4$:A (A = Mn, lanthanide) ; $3Ca_3(PO_4)_2$.Ca(F,Cl)$_2$:Sb,Mn ; $CaSiO_3$:Mn,Pb ; $Ca_2Al_2Si_2O_7$:Ce ; (Ca,Mg)$SiO_3$:Ce ; (Ca,Mg)$SiO_3$:Ti ; 2SrO.6($B_2O_3$).$SrF_2$:Eu ; $3Sr_3(PO_4)_2$.$CaCl_2$:Eu ; $A_3(PO_4)_2$.$ACl_2$:Eu (A = Sr, Ca, Ba) ; (Sr,Mg)$_2P_2O_7$:Eu ; (Sr,Mg)$_3(PO_4)_2$:Sn ; SrS:Ce ; SrS:Sm,Ce ; SrS:Sm ; SrS:Eu ; SrS:Eu, Sm ; SrS:Cu, Ag ; $Sr_2P_2O_7$:Sn ; $Sr_2P_2O_7$:Eu; $Sr_4Al_{14}O_{25}$:Eu ; $SrGa_2S_4$:A (A = lanthanide, Pb) ; $SrGa_2S_4$:Pb ; $Sr_3Gd_2Si_6O_{18}$:Pb, Mn ; $YF_3$:Yb,Er ; $YF_3$:Ln (Ln = lanthanide) ; $YLiF_4$:Ln (Ln = lanthanide) ; $Y_3Al_5O_12$:Ln (Ln = lanthanide) ; $YAl_3(BO_4)_3$:Nd,Yb ; (Y,Ga)$BO_3$:Eu ; (Y,Gd)$BO_3$:Eu ; $Y_2Al_3Ga_2O_{12}$:Tb $Y_2SiO_5$:Ln (Ln = lanthanide) ; $Y_2O_3$:Ln (Ln = lanthanide) ; $Y_2O_2S$:Ln (Ln = lanthanide) ; $YVO_4$:A (A = lanthanide, In) ; Y(P,V)$O_4$:Eu ; $YTaO_4$:Nb ; $YAlO_3$:A (A = Pr, Tm, Er, Ce) ; YOCl:Yb,Er ; $LnPO_4$:Ce,Tb (Ln = lanthanide ou des mélanges de lanthanides) ; $LuVO_4$:Eu ; $GdVO_4$:Eu ; $Gd_2O_2S$:Tb ; $GdMgB_5O_{10}$:Ce, Tb ; LaOBr:Tb ; $La_2O_2S$:Tb ; $LaF_3$:Nd,Ce ; $BaYb_2F_8$:Eu ; $NaYF_4$:Yb,Eu ; $NaGdF_4$:Yb, Er ; $NaLaF_4$:Yb,Er ; $LaF_3$:Yb,Er,Tm ; $BaYF_5$:Yb,Er ; $Ga_2O_3$:Dy ; GaN:A (A = Pr, Eu, Er, Tm) ; $Bi_4Ge_3O_{12}$ ; $LiNbO_3$:Nd,Yb ; $LiNbO_3$:Er ; $LiCaAlF_6$:Ce ; $LiSrAlF_6$:Ce ; $LiLuF_4$:A (A = Pr, Tm, Er, Ce) ; $Li_2B_4O_7$:Mn ; $SiO_x$:Er,Al ($0 \leq x \leq 2$).

15. Analyse suivant l'une des revendications précédentes, **caractérisée en ce que** l'on utilise comme matériau pour les nanoparticules dil, $YVO_4$:Eu, $YVO_4$:Sm, $YVO_4$:Dy, $LaPO_4$:Eu, $LaPO_4$:Ce, $LaPO_4$:Ce,Tb, $LaPO_4$:Ce,Dy, $LaPO_4$:Ce,Nd, ZnS:Tb, ZnS:Tb$F_3$, ZnS:Eu, ZnS:Eu$F_3$, $Y_2O_3$:Eu, $Y_2O_2S$:Eu, $Y_2SiO_5$:Eu ; $SiO_2$:Dy ; $SiO_2$:Al, $Y_2O_3$:Tb, CdS:Mn, ZnS:Tb, ZnS:Ag ou ZnS:Cu.

16. Analyse suivant l'une des revendications précédentes, **caractérisée en ce qu'**un matériau avec une structure cristalline cubique du réseau hôte est utilisé pour les nanoparticules dil.

17. Analyse suivant l'une des revendications précédentes, **caractérisée en ce que** l'on utilise comme matériau pour les nanoparticules dil, $MgF_2$:Mn, ZnS:Mn, ZnS:Ag, ZnS:Cu, $CaSiO_3$:Ln, CaS:Ln, CaO:Ln, ZnS:Ln, $Y_2O_3$:Ln ou $MgF_2$:Ln (Ln = lanthanides).

18. Analyse suivant l'une des revendications précédentes, **caractérisée en ce que** le donneur et/ou l'accepteur comprennent des nanoparticules dil, qui émettent le rayonnement électromagnétique avec un déplacement Stokes ou anti-Stokes après une excitation énergétique avec un rayonnement électromagnétique avec des longueurs d'onde dans le domaine de la lumière infrarouge, de la lumière visible, de l'U.V., la lumière X ou le rayonnement $\gamma$ ou un rayonnement particulaire comme une rayonnement électronique.

19. Analyse suivant l'une des revendications précédentes, **caractérisée en ce que** le donneur comprend des nanoparticules dil et l'accepteur consiste en un matériau conducteur, de préférence un métal, en particulier l'or, l'argent ou le platine, ou un oxyde conducteur, en particulier l'oxyde d'indium-étain (ITO)
ou un polymère conducteur, qui se présente de préférence, sous forme particulaire comme des nano- ou microparticules ou comme une surface plane, le cas échéant structurée.

20. Analyse suivant l'une des revendications précédentes, **caractérisée en ce qu'**il s'agit d'une analyse homogène sans étape de lavage ou de séparation.

21. Analyse suivant l'une des revendications précédentes, **caractérisée en ce que** l'analyse est un immunodosage homogène pour la détection d'au moins un analyte, en particulier au moins un anticorps monoclonal ou polyclonal, des protéines, des peptides, des oligonucléotides, des acides nucléiques, des oligo- ou polysaccharides, des haptènes ou un antigène synthétique ou naturel, de faible poids moléculaire, dans un échantillon, en particulier des frottis, des expectorations, des ponctions d'organe, des biopsies, des sécrétions, des liqueurs, la bile, le sang, le fluide lymphatique, l'urine et/ou les fécès.

22. Analyse suivant l'une des revendications précédentes, **caractérisée en ce que** la surface de la ou des nanoparticules dil est préparée de sorte que des molécules d'affinité peuvent y être couplées.

23. Analyse suivant la revendication 22, **caractérisée en ce que** la surface des nanoparticules dil est chimiquement modifiée et/ou présente des groupes réactifs et/ou des molécules de liaison liées de manière covalente ou non covalente, où les molécules de liaison liées peuvent présenter pour leur part, des groupes réactifs.

24. Analyse suivant la revendication 23, **caractérisée en ce que** les groupes réactifs sont des radicaux amino, acide carboxylique, thiol, thioéther, disulfure, imidazole, guanidine, hydroxyle, indole, diols vicinaux, aldéhyde, alpha-haloacétyle, N-maléimide, mercuro-organyle, arylhalogénure, anhydride d'acide, isocyanate, isothiocyanate,

halogénure d'acide sulfonique, imidoester, diazoacétate, sel de diazonium, 1,2-dicétone, carbonyle $\alpha,\beta$-insaturé, acide phosphonique, ester d'acide phosphorique, acide sulfonique, azolide ou des dérivés des radicaux cités.

**25.** Analyse suivant la revendication 23 ou 24, **caractérisée en ce que** les molécules de liaison sont des molécules d'acide nucléique, des dérivés d'acide phosphonique, l'éthylèneglycol, des alcools primaires, des dérivés d'amine, des polymères ou copolymères, des réactifs de couplage polymérisables, des enveloppes en silice et des molécules de type chaîne avec une polarité opposée à celle de la surface des nanoparticules dil.

**26.** Analyse suivant la revendication 25, **caractérisée en ce que** les réactifs de couplage polymérisable sont le diacétylène, le styrène-butadiène, l'acétate de vinyle, un acrylate, l'acrylamide, un vinyle, un styrène, l'oxyde de silicium, l'oxyde de bore, l'oxyde de phosphore, un borate, le pyrrole, un polypyrrole et un phosphate ainsi que des polymères d'au moins certains des agents mentionnés.

**27.** Analyse suivant la revendication 22 à 26, **caractérisée en ce que** la molécule d'affinité est une protéine, un peptide, un oligonucléotide ou d'autres molécules d'acide nucléique ou molécules de type acide nucléique, comme les PNA ou morpholino, des oligo- ou polysaccharide, un haptène, comme la biotine ou la digoxine ou des antigènes synthétique ou naturels de faible poids moléculaire ou des épitopes.

**28.** Analyse suivant la revendication 22 à 27, **caractérisée en ce que** les molécules d'affinité peuvent interagir avec les molécules cible.

**29.** Analyse suivant la revendication 28, **caractérisée en ce que** la molécule cible est une enzyme, un anticorps, une molécule liant l'acide nucléique, un acide nucléique, un polynucléotide ou morpholino.

**30.** Analyse suivant la revendication 29, **caractérisée en ce que** l'enzyme est une endonucléase, une protéase, une kinase, une phosphatase ou un acide aminé ou une enzyme modifiant ou clivant l'acide nucléique.

**31.** Analyse suivant l'une des revendications 1 à 30, **caractérisée en ce que** les groupes moléculaires A et B sont les constituants d'une et même molécule.

**32.** Analyse suivant l'une des revendications précédentes, **caractérisée en ce que** les groupes moléculaires A et B sont couplés à la même molécule d'affinité.

**33.** Analyse suivant la revendication 31, **caractérisée en ce qu'**une interaction de la molécule d'affinité avec la molécule cible a pour conséquence une modification de la distance spatiale des groupes moléculaires A et B.

**34.** Analyse suivant l'une des revendications précédentes, **caractérisée en ce qu'**elle est utilisée pour la quantification des acides nucléiques.

**35.** Analyse suivant l'une des revendications 1 à 30, **caractérisée en ce que** les groupes moléculaires A et B sont les constituants de molécules différentes.

**36.** Analyse suivant la revendication 35, **caractérisée en ce que** les groupes moléculaires A et B sont couplés chaque fois à une molécule d'affinité propre.

**37.** Analyse suivant la revendication 36, **caractérisée en ce que** les molécules d'affinité attribuées aux groupes moléculaires A et B peuvent interagir spécifiquement avec la même molécule cible.

**38.** Analyse suivant la revendication 37, **caractérisée en ce que** les molécules d'affinité attribuées aux groupes moléculaires A et B peuvent interagir spécifiquement l'une avec l'autre.

**39.** Analyse suivant la revendication 37 ou 38, **caractérisée en ce qu'**une interaction des molécules d'affinité attribuées aux groupes moléculaires A et B avec la molécule cible commune ou l'une avec l'autre a pour conséquence, une modification de la distance spatiale des groupes moléculaires A et B.

**40.** Procédé de détection d'une molécule cible, contenant les étapes de

- préparation d'une analyse suivant une des revendications 1 à 34, où dans l'analyse, les groupes moléculaires

A et B sont les constituants d'une et même molécule et couplent la même molécule d'affinité, qui peut interagir avec une molécule cible spécifique et une telle interaction réalise une modification de la distance entre les groupes moléculaires A et B,
- addition d'un échantillon contenant la molécule cible pour l'analyse,
- excitation de l'analyse avec l'échantillon par une source de rayonnement électromagnétique ou particulaire,
- mesure du rayonnement électromagnétique émis par l'analyse avec l'échantillon,

où i'intensité ou le spectre du rayonnement électromagnétique émis ou le développement temporel de l'émission du rayonnement électromagnétique est une mesure de la quantité de molécule cible dans l'échantillon.

**41.** Procédé de détection d'une molécule cible, contenant les étapes de

- préparation d'une analyse suivant une des revendications 1 à 30 ou 35 à 39, où les groupes moléculaires A et B sont les constituants de molécules différentes et les molécules d'affinité attribuées aux groupes moléculaires A et B peuvent interagir spécifiquement avec une et même molécule cible ou les molécules d'affinité attribuées aux groupes moléculaires A et B peuvent interagir spécifiquement l'une avec l'autre, et dans les deux cas, une interaction entraîne une modification de la distance entre les groupes moléculaires A et B,
- addition d'un échantillon contenant la molécule cible pour l'analyse,
- excitation de l'analyse avec l'échantillon par une source de rayonnement électromagnétique ou particulaire,
- mesure du rayonnement électromagnétique émis par l'analyse avec l'échantillon,

où l'intensité ou le spectre du rayonnement électromagnétique émis ou le développement temporel de l'émission du rayonnement électromagnétique est une mesure de la quantité de molécule cible dans l'échantillon.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3996345 A
- US 4160016 A
- US 4174384 A
- US 4199559 A
- US 4996143 A
- US 5532129 A
- US 5565332 A
- US 5538848 A
- US 5723591 A
- US 5210015 A
- US 5312728 A
- US 5491084 A
- US 5625048 A
- WO 8707955 A
- EP 242527 A
- EP 439036 A
- WO 9201225 A
- US 4822733 A
- US 5279943 A
- US 5622821 A
- US 5656433 A
- US 5998146 A
- US 6239271 B
- WO 0029617 A
- US 6207392 B1
- US 5043265 A
- US 5674698 A
- WO 9843072 A
- US 5893999 A
- US 6159686 A


### In der Beschreibung aufgeführte Nicht-Patentliteratur


- **B. J. R. LAKOWICZ.** Principles of Fluorescence Spectroscopy. Academic Press, 1999, 368-445
- **YAMAMOTO et al.** *J. Mol. Biol.,* 1994, vol. 241, 714-731
- **BOISCLAIR et al.** *J. of Biomolecular Screening,* 2000, vol. 5, 319-328
- **HEYDUK et al.** *SPIE,* 1998, vol. 3256, 218-222
- **LAKOWICZ et al.** *Biophys. Chem.,* 1990, vol. 36, 99-115
- **K. CAI et al.** *J. Biol. Chem.,* 1996, vol. 271, 27311-27320
- **HOCHSTRASSER et al.** *Biophys. Chem.,* 1992, vol. 45, 133-141
- **OZAKI et al.** *Nucl. Acids Res.,* 1992, vol. 20, 5205-5214
- **S. WANG et al.** *Biochemistry,* 1988, vol. 27, 2033-2039
- **HOLLAND et al.** *Proc. Natl. Acad. Sci USA,* 1991, vol. 88, 7276-7280
- **LEE et al.** *Nuleic Acids Res.,* 1993, vol. 21, 3761-3766
- **TYAGI ; KRAMER.** *Nature Biotechnology,* 1996, vol. 14, 303-306
- **POLLOK ; HEIM.** Übersichtsartikel. *Trends Cell Biol.,* 1999, vol. 9, 57-60
- **SELVIN.** *IEEE J. of Selected Topics in Quantum Electronics,* 1996, vol. 2, 1077-1087
- **CLARK et al.** *Anal. Biochem.,* 1993, vol. 210, 1 ff
- **THOMAS et al.** *Proc. Natl. Acad. Sci.,* 1978, vol. 75, 5746 ff
- **S. G. JONES et al.** *Journal of Fluorescence,* 2001, vol. 11, 13-21
- **TANAKA et al.** *J. Photochem. Photobiol.,* 1993, vol. 74, 15 ff
- **MATKO et al.** *Biochemistry,* 1992, vol. 31, 703 ff
- **BAWENDI ; C. KAGAN et al.** *Phys. Rev. Lett.,* 1996, vol. 76, 1517-1520
- **O. SCHMELZ et al.** *Langmuir,* 2001, vol. 17, 2861-2865
- **XIAOGANG PENG et al.** *J. Am. Chem. Soc.,* 1997, vol. 119, 7019-7029
- **K. RIWOTZKI et al.** Angewandte Chemie, Int. 2001, vol. 40, 573-576
- **K. RIWOTZKI ; M. HAASE.** *Journal of Physical Chemistry B,* 1998, vol. 102, 10129-10135
- **H. MEYSSAMY ; K. RIWOTZKI ; A. KORNOWSKI ; S. NAUSED ; M. HAASE.** *Advanced Materials,* 1999, vol. 11 (10), 840-844
- **K. RIWOTZKI ; H. MEYSSAMY ; A. KORNOWSKI ; M. HAASE.** *Journal of Physical Chemistry B,* 2000, vol. 104, 2824-2828
- **Q. LI ; L. GAO ; D. S. YAN.** *Nanostructured Materials,* 1999, vol. 8, 825 ff
- **M. YIN ; W. ZHANG ; S. XIA ; J. C. KRUPA.** *Journal of Luminescence,* vol. 68

- **Y. H. LI ; C. M. MO ; L. D. ZHANG ; R. C. LIU ; Y. S. LIU.** *Nanostructured Materials,* 1999, vol. 11 (3), 307-310
- **Y. L. SOO ; S. W. HUANG ; Z. H. MING ; Y. H. KAO ; G. C. SMITH ; E. GOLDBURT ; R. HODEL ; B. KULKARNI ; J. V. D. VELIADIS ; R. N. BHARGAVA.** *Journal of Applied Physics,* 1998, vol. 83 (10), 5404-5409
- **R. N. BHARGAVA ; D. GALLAGHER ; X. HONG ; A. NURMIKKO.** *Physical Review Letters,* 1994, vol. 72, 416-419
- **R. N. BHARGAVA ; D. GALLAGHER ; T. WELKER.** *Journal of Luminescence,* 1994, vol. 60, 275 ff
- Luminescent Materials: 1. Inorganic Phosphors. Ullmann's Encyclopedia of Industrial Chemistry. WILEY-VCH, 2001
- **D. DEXTER.** *J. Chem. Phys.,* 1953, vol. 21, 836-850
- **T. JÜSTEL et al.** Angewandte Chemie. 1998, vol. 37, 3084-3103
- **NIEDBALA et al.** *Analytical Biochemistry,* 2001, vol. 293, 22-30
- **MOEDRITZER ; IRANI.** *J. Org. Chem.,* 1966, vol. 31, 1603
- **R. P. HAUGLUND.** Handbook of Fluorescent Probes and Research Chemicals. Molecular Probes, Inc, 2001
- **GREG T. HERMANSON.** Bioconjugate Techniques. Academic Press, 1996
- **K. RIWOTZKI ; M. HAASE.** *Journal of Physical Chemistry B,* 1998, vol. 102, 10130
- **H. MEYSSAMY ; K. RIWOTZKI ; A. KORNOWSKI ; S. NAUSED ; M. HAASE.** *Advanced Materials,* 1999, vol. 11 (10), 843